# EUROPEAN PATENT SPECIFICATION

(11) **EP 2 663 288 B1**
(45) Date of publication and mention of the grant of the patent: **21.12.2022**
(21) Application number: 12733900.0
(22) Date of filing: 13.01.2012
(51) Int. Cl.: A61K 9/127, A61K 9/19, A61P 31/12, A61P 37/04, A61K 39/00, A61K 39/12, A61K 39/145

(54) **METHODS FOR PREPARING VESICLES AND FORMULATIONS PRODUCED THEREFROM**
VERFAHREN ZUR VESIKELHERSTELLUNG UND DARIN HERGESTELLTE FORMULIERUNGEN
PROCÉDÉ POUR LA PRÉPARATION DE VÉSICULES ET FORMULATIONS PRODUITES À PARTIR DE CELLES-CI

(30) Priority: 13.01.2011 US 201161432569 P
(43) Date of publication of application: 20.11.2013
(73) Proprietor: Variation Biotechnologies Inc., Ottawa, ON K1V 1C1 (CA)
(72) Inventor: ANDERSON, David, E., Boston MA 02116 (US); PERRIE, Yvonne, West Midlands B94 6QY (GB); WILKHU, Jitinder Singh, West Midlands B11 3DF (GB); KIRCHMEIER, Marc, Harleysville PA 19438 (US)
(74) Representative: Greenwood, Matthew David
(86) International application number: PCT/US2012/021389
(87) International publication number: WO 2012/097347

(56) References cited:
- WO-A1-88/06882
- WO-A1-2010/033812
- US-A- 5 160 669
- US-A- 5 250 236
- US-A- 5 340 588
- US-A- 5 567 434
- US-A- 5 679 355
- US-A- 5 879 703
- US-A1- 2008 213 461
- US-A1- 2010 226 932
- SACHA GNJATIC ET AL: "TLR Agonists", THE CANCER JOURNAL, vol. 16, no. 4, 1 July 2010 (2010-07-01), pages 382-391, XP055112514, ISSN: 1528-9117, DOI: 10.1097/PPO.0b013e3181eaca65
- M. COLLINS ET AL: "NON-IONIC SURFACTANT VESICLE FORMULATION OF STIBOGLUCONATE FOR CANINE LEISHMANIASIS", JOURNAL OF PHARMACY AND PHARMACOLOGY, vol. 42, no. S1, 12 December 1990 (1990-12-12), pages 53P-53P, XP055334525, LONDON; GB ISSN: 0022-3573, DOI: 10.1111/j.2042-7158.1990.tb14426.x

## Description

### Cross Reference to Related Applications

This application claims priority to United States Provisional Patent Application Serial Number 61/432,569, filed January 13 2011.

### Background

Vesicles were first described in the 1960s as a model of cellular membranes (see Bangham et al., J. Mol. Biol. 13:238-252, 1965). Vesicles have found a number of applications in the delivery of small molecule drugs, vaccine adjuvancy, gene transfer and diagnostic imaging (e.g., see Liposome Technology, 3rd Edition, Edited by Gregory Gregoriadis, Informa HealthCare, 2006 and Liposomes: A Practical Approach (The Practical Approach Series, 264), 2nd Edition, Edited by Vladimir Torchilin and Volkmar Weissig, Oxford University Press, USA, 2003).

A number of methods for preparing vesicles have been described (e.g., see references cited above and Walde and Ichikawa, Biomol. Eng., 18:143-177, 2001). However, there remains a need in the art for methods that can be used to entrap substances within vesicles.

One method that has been described in the art is the so-called melt method. Vesicle-forming lipids are initially melted at high temperatures (e.g., 120°C). An emulsion is created in a second step by adding an aqueous buffer (e.g., bicarbonate buffer) to the molten lipids. Finally, the substance to be entrapped is homogenized with the components of the emulsion at a reduced temperature (e.g., 50°C) prior to lyophilization. Alternatively, vesicles from the emulsion are lyophilized and then reconstituted in the presence of the substance to be entrapped.

Patent WO 2010/033812 A1 discloses in a three step melt method, including melting of a 5:4:1 molar ratio of MPG, CHO and DCP lipid mixture at 120°C. An emulsion is formed by homogenizing at 50°C with an aqueous phase. Antigen solution is added at this step; thus, implicitly antigen solution was at 50°C. After cooling to 30°C, the product was mixed for 2 hours and lyophilized.

While methods such as this one may well be suitable for entrapping substances that can withstand high temperatures and/or small molecules that are able to diffuse rapidly into empty vesicles we have found that they are unsuitable for entrapping the types of antigens (e.g., polypeptides, viruses, etc.) that are commonly involved in vaccines. In particular, we have found that these methods produce low entrapment efficiencies and can dramatically reduce the activity of the underlying antigen (e.g., as measured by immune responses). There is therefore a need in the art for methods of preparing vesicles that are capable of entrapping antigens while minimizing impact on antigen activity.

### Summary

The present invention provides a method comprising: providing a molten mixture of vesicle-forming lipids, the mixture consisting of monopalmitoyl glycerol (MPG), cholesterol (CHO) and dicetyl phosphate (DCP), at a ratio of 5:4:1 wherein the molten mixture of vesicle-forming lipids is melted at a temperature of less than 120°C; and adding the molten mixture of vesicle-forming lipids to an aqueous solution comprising an antigen at a temperature of less than 120°C, said antigen consisting of an attenuated virus or an inactivated virus, and a suitable buffer, wherein said aqueous solution is at a temperature of 30°C to 35°C, and homogenizing the resulting mixture at 30°C to 35°C such that antigen-containing vesicles are formed, wherein said vesicles comprise an aqueous core enclosed by one or more lipid bilayers and preferably further adding a lyoprotectant to the vesicles.

### Brief Description of the Drawing

**Figure 1** shows a DSC thermogram of DCP, MPG and CHO (cholesterol). Their melting onset transitions are overlayed onto a single scan. The DSC thermogram for a mixture of these lipids is also shown.
**Figure 2** shows a freeze fracture image of NISVs showing a large sliced vesicle above an untouched vesicle. The scale bar in the lower left hand corner represents 0.5 µm.
**Figure 3** shows Langmuir monolayer isotherms representing surface pressure as a function of mean molecular area representing individual surfactants and a mixed formulation in the ratio 5:4:1 of MPG:CHO:DCP.
**Figures 4A** and **4B** shows the effect of trypsin digestion on NISVs retention of radioactive-labelled H1N1 antigen, formulated by four different methods.
**Figure 5** shows microscopic analysis by cryo-TEM of NISVs formulated by four different methods. (A) Inverted melt method; (B) melt method; (C) melt method- lower temperature of antigen addition; and (D) liposomal chloroform method.

### Definitions

Throughout the present disclosure, several terms are employed that are defined in the following paragraphs.

As used herein, the term "antigen" refers to a substance containing one or more epitopes (either linear, conformational or both) that can be recognized by an antibody. An antigen can be a virus, a polypeptide, a polynucleotide, a polysaccharide, etc The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes. An antigen may be an "immunogen. In the present invention, an antigen consists of an attenuated virus or an inactivated virus.

As used herein, the term "entrapping" refers to any kind of physical association between a substance and a vesicle, e.g., encapsulation, adhesion (to the inner or outer wall of the vesicle) or embedding in the wall with or without extrusion of the substance. The term is used interchangeably with the terms "loading" and "containing".

As used herein, the terms "immune response" refer to a response elicited in an animal. An immune response may refer to cellular immunity, humoral immunity or may involve both. An immune response may also be limited to a part of the immune system. For example, in certain embodiments, an immunogenic formulation may induce an increased IFNγ response. In certain embodiments, an immunogenic formulation may induce a mucosal IgA response (e.g., as measured in nasal and/or rectal washes). In certain embodiments, an immunogenic formulation may induce a systemic IgG response (e.g., as measured in serum).

As used herein, the term "immunogenic" means capable of producing an immune response in a host animal against a non-host entity (e.g., an influenza virus). In certain embodiments, this immune response forms the basis of the protective immunity elicited by a vaccine against a specific infectious organism (e.g., an influenza virus). An "immunogen" is an immunogenic substance.

As used herein, the terms "therapeutically effective amount" refer to the amount sufficient to show a meaningful benefit in a patient being treated. The therapeutically effective amount of an immunogenic formulation may vary depending on such factors as the desired biological endpoint, the nature of the formulation, the route of administration, the health, size and/or age of the patient being treated, etc.

As used herein, the term "polypeptide" or "protein" refers to a polymer of amino acids. In some embodiments, polypeptides may include moieties other than amino acids (e.g., may be glycoproteins, proteoglycans, lipoproteins, etc.) and/or may be otherwise processed or modified. Those of ordinary skill in the art will appreciate that a "protein" can be a complete polypeptide chain as produced by a cell (with or without a signal sequence), or can be a portion thereof. Those of ordinary skill will appreciate that a protein can sometimes include more than one polypeptide chain, for example linked by one or more disulfide bonds or associated by other means. Polypeptides may contain L-amino acids, D-amino acids, or both and may contain any of a variety of amino acid modifications or analogs known in the art. Useful modifications include, e.g., terminal acetylation, amidation, etc. In some embodiments, polypeptides may comprise natural amino acids, non-natural amino acids, synthetic amino acids, and combinations thereof. In certain embodiments a polypeptide may include at least 50 amino acids, at least 75 amino acids, at least 100 amino acids, at least 150 amino acids, at least 250 amino acids or at least 500 amino acids.

As used herein, the term "polysaccharide" refers to a polymer of sugars. The polymer may include natural sugars (e.g., arabinose, lyxose, ribose, xylose, ribulose, xylulose, allose, altrose, galactose, glucose, gulose, idose, mannose, talose, fructose, psicose, sorbose, tagatose, mannoheptulose, sedoheptulose, octolose, and sialose) and/or modified sugars (e.g., 2'-fluororibose, 2'-deoxyribose, and hexose). Exemplary polysaccharides include starch, glycogen, dextran, cellulose, etc.

As used herein, the term "polynucleotide" refers to a polymer of nucleotides. The polymer may include natural nucleosides (i.e., adenosine, thymidine, guanosine, cytidine, uridine, deoxyadenosine, deoxythymidine, deoxyguanosine, and deoxycytidine), nucleoside analogs (e.g., 2-aminoadenosine, 2-thiothymidine, inosine, pyrrolo-pyrimidine, 3-methyl adenosine, 5-methylcytidine, C5-bromouridine, C5-fluorouridine, C5-iodouridine, C5-propynyl-uridine, C5-propynyl-cytidine, C5-methylcytidine, 7-deazaadenosine, 7-deazaguanosine, 8-oxoadenosine, 8-oxoguanosine, O(6)-methylguanine, 4-acetylcytidine, 5-(carboxyhydroxymethyl)uridine, dihydrouridine, methylpseudouridine, I-methyl adenosine, 1-methyl guanosine, N6-methyl adenosine, and 2-thiocytidine), chemically modified bases, biologically modified bases (e.g., methylated bases), intercalated bases, modified sugars (e.g., 2'-fluororibose, ribose, 2'-deoxyribose, 2'-O-methylcytidine, arabinose, and hexose), or modified phosphate groups (e.g., phosphorothioates and 5' -N-phosphoramidite linkages).

As used herein, the term "small molecule therapeutic" refers to a non-polymeric therapeutic molecule that may contain several carbon-carbon bonds and have a molecular weight ofless than about 1500 Da (e.g., less than about 1000 Da, less than about 500 Da or less than about 200 Da). A small molecule therapeutic can be synthesized in a laboratory (e.g., by combinatorial synthesis, using an engineered microorganism, etc.) or can be found in nature (e.g., a natural product). In general, a small molecule therapeutic may alter, inhibit, activate, or otherwise affect a biological event. For example, small molecule therapeutics may include, but are not limited to, anti-AIDS substances, anti-cancer substances, antibiotics, anti-diabetic substances, immunosuppressants, anti-viral substances, enzyme inhibitors, neurotoxins, opioids, hypnotics, anti-histamines, lubricants, tranquilizers, anti-convulsants, muscle relaxants and anti-Parkinson substances, anti-spasmodics and muscle contractants including channel blockers, miotics and anti-cholinergics, anti-glaucoma compounds, anti-parasite and/or anti-protozoa! compounds, modulators of cell-extracellular matrix interactions including cell growth inhibitors and anti-adhesion molecules, vasodilating agents, inhibitors of DNA, RNA or protein synthesis, anti-hypertensives, analgesics, anti-pyretics, steroidal and non-steroidal anti-inflammatory agents, anti-angiogenic factors, anti-secretory factors, anticoagulants and/or anti-thrombotic agents, local anesthetics, ophthalmics, prostaglandins, anti-depressants, anti-psychotic substances, anti-emetics, and imaging agents. A more complete listing of exemplary small molecules suitable for use in the methods of the present disclosure may be found in Pharmaceutical Substances: Syntheses, Patents, Applications, Edited by Axel Kleemann and Jurgen Engel, Thieme Medical Publishing, 1999; Merck Index: An Encyclopedia of Chemicals, Drugs, and Biologicals, Edited by Susan Budavari et al., CRC Press, 1996, and the United States Pharmacopeia-25/National formulary-20, published by the United States Pharmacopeial Convention, Inc., 2001. Preferably, though not necessarily, the small molecule is one that has already been deemed safe and effective for use by the appropriate governmental agency or body. For example, drugs for human use listed by the FDA under 21 C.F.R. §§ 330.5, 331 through 361,and 440 through 460 and drugs for veterinary use listed by the FDA under 21 C.F.R. §§ 500 through 589, are all considered acceptable for use in accordance with the methods of the present disclosure.

As used herein, the term "treat" (or "treating", "treated", "treatment", etc.) refers to the administration of a formulation to a patient who has a disease, a symptom of a disease or a predisposition toward a disease, with the purpose to alleviate, relieve, alter, ameliorate, improve or affect the disease, a symptom or symptoms of the disease, or the predisposition toward the disease. In certain embodiments, the term "treating" refers to the vaccination of a patient.

### Detailed Description of Certain Embodiments

### I. Methods for Preparing Vesicles - Inverted Melt

The present invention provides a method for preparing vesicles. Vesicles generally have an aqueous compartment enclosed by one or more bilayers which include lipids, optionally with other molecules. For example, as discussed in more detail below, in some embodiments, the vesicles of the present disclosure comprise transport enhancing molecules (e.g., bile acids or salts thereof) which facilitate the transport of lipids across mucosal membranes.

In certain embodiments the antigen is thermolabile.

In certain embodiments, the method further comprises a step of adding an adjuvant after the antigen-containing vesicles are formed, wherein the adjuvant is a TLR-3 agonist.

In certain embodiments, the molten mixture of vesicle-forming lipids comprises an adjuvant, wherein the adjuvant is a TLR-4 agonist.

In certain embodiments, the method further comprises a step of lyophilizing a formulation that comprises the antigen-containing vesicles.

The method of the present invention avoid exposing antigen to organic solvents and high temperatures. Without wishing to be limited to any theory, this may explain the high activity (i.e., antigenicity and/or immunogenicity) of the entrapped antigens in the resulting formulations.

### Vesicle-forming lipids

Lipids are organic molecules that are generally insoluble in water but soluble in nonpolar organic solvents (e.g., ether, chloroform, acetone, benzene, etc.). Fatty acids are one class of lipids that include an acid moiety linked to a saturated or unsaturated hydrocarbon chain. Specific examples include lauric acid, palmitic acid, stearic acid, arachidic acid, palmitoleic acid, oleic acid, linoleic acid, linolenic acid, arachidonic acid, etc. Alkali metal salts of fatty acids are typically more soluble in water than the acids themselves. Fatty acids and their salts that include hydrocarbon chains with eight or more carbons often exhibit amphiphilic properties due to the presence of both hydrophilic (head) and hydrophobic (tail) regions in the same molecule. Non-ionic lipids that include polar head groups can also exhibit amphiphilic (i.e., surfactant) properties. The triesters of fatty acids with glycerol (1,2,3-trihydroxypropane) compose another class of lipids known as triglycerides that are commonly found in animal fats and plant oils. Esters of fatty acids with long chain monohydric alcohols form another class of lipids that are found in waxes. Phospholipids are yet another class of lipids. They resemble the triglycerides in being ester or amide derivatives of glycerol or sphingosine with fatty acids and phosphoric acid. The phosphate moiety of the resulting phosphatidic acid may be further esterified with ethanolamine, choline or serine in the phospholipid itself. It is to be understood that the methods may be used with any lipid that is capable of forming vesicles including any of the lipids that are described in the prior art (e.g., in Liposome Technology, 3rd Edition, Edited by Gregory Gregoriadis, Informa HealthCare, 2006 and Liposomes: A Practical Approach (The Practical Approach Series, 264), 2nd Edition, Edited by Vladimir Torchilin and Volkmar Weissig, Oxford University Press, USA, 2003).

In some embodiments, the vesicle-forming lipid is a phospholipid. Any naturally occurring or synthetic phospholipid can be used. Without limitation, examples of specific phospholipids are L-a-(distearoyl) lecithin, L-a-(diapalmitoyl) lecithin, L-a-phosphatide acid, L-a-(dilauroyl)-phosphatidic acid, L-a (dimyristoyl) phosphatidic acid, L-a(dioleoyl)phosphatidic acid, DL-a(dipalmitoyl) phosphatidic acid, L-a(distearoyl) phosphatidic acid, and the various types of L-a-phosphatidylcholines prepared from brain, liver, egg yolk, heart, soybean and the like, or synthetically, and salts thereof.

In some embodiments, the vesicle-forming lipid is a non-ionic surfactant. Non-ionic surfactant vesicles are referred to herein as "NISVs". Without limitation, examples of suitable non-ionic surfactants include ester-linked surfactants based on glycerol. Such glycerol esters may comprise one of two higher aliphatic acyl groups, e.g., containing at least ten carbon atoms in each acyl moiety. Surfactants based on such glycerol esters may comprise more than one glycerol unit, e.g., up to 5 glycerol units. Glycerol monoesters may be used, e.g., those containing a C₁₂-C₂₀alkanoyl or alkenoyl moiety, for example caproyl, lauroyl, myristoyl, palmitoyl, oleyl or stearoyl. An exemplary non-ionic surfactant is 1-monopalmitoyl glycerol.

In some embodiments, ether-linked surfactants may also be used as the non-ionic surfactant. For example, ether-linked surfactants based on glycerol or a glycol having a lower aliphatic glycol of up to 4 carbon atoms, such as ethylene glycol, are suitable. Surfactants based on such glycols may comprise more than one glycol unit, e.g., up to 5 glycol units (e.g., diglycolcetyl ether and/or polyoxyethylene-3-lauryl ether). Glycol or glycerol monoethers may be used, including those containing a C₁₂-C₂₀ alkanyl or alkenyl moiety, for example capryl, lauryl, myristyl, cetyl, oleyl or stearyl. Ethylene oxide condensation products that can be used include those disclosed in PCT Publication No. WO88/06882 (e.g., polyoxyethylene higher aliphatic ether and amine surfactants). Exemplary ether-linked surfactants include 1-monocetyl glycerol ether and diglycolcetyl ether.

### Other components

In some embodiments, the vesicles may contain other lipid and non-lipid components, as long as these do not prevent vesicle formation. It is to be understood that these components may be co-mixed with the vesicle-forming lipids and/or may be co-mixed with the antigen(s). In some embodiments, we have found that it can be advantageous to co-mix these components with the vesicle-forming lipids.

In some embodiments, the vesicles may include a transport enhancing molecule which facilitates the transport of lipids across mucosal membranes. As described in U.S. Patent No. 5,876,721, a variety of molecules may be used as transport enhancers. For example, cholesterol derivatives in which the C₂₃ carbon atom of the side chain carries a carboxylic acid, and/or derivatives thereof, may be used as transport enhancers. Such derivatives include, but are not limited to, the "bile acids" cholic acid and chenodeoxycholic acid, their conjugation products with glycine or taurine such as glycocholic and taurocholic acid, derivatives including deoxycholic and ursodeoxycholic acid, and salts of each of these acids. **NISVs** that further include a bile acid or salt are referred to herein as "bilosomes". In some embodiments, transport enhancers include acyloxylated amino acids, such as acylcarnitines and salts thereof. For example, acylcarnitine containing C₆_20 alkanoyl or alkenoyl moieties, such as palmitoylcarnitine, may be used as transport enhancers. As used herein, the term acyloxylated amino acid is intended to cover primary, secondary and tertiary amino acids as well as *a,* p, and y amino acids. Acylcarnitines are examples of acyloxylated y amino acids. It is to be understood that vesicles may comprise more than one type of transport enhancer, e.g., one or more different bile salts and one or more acylcarnitines. The transport enhancer(s), if present, will typically comprise between 1 and 400% percent by weight of the vesicle-forming lipid (e.g., the non-ionic surfactant). In some embodiments, the transport enhancer(s), if present will comprise between 1 and 40% percent by weight of the vesicle-forming lipid (e.g., between 1 and 20% by weight, between 1 and 25% by weight, between 1 and 30% by weight, between 1 and 35% by weight, between 2 and 25% by weight, between 2 and 30% by weight or between 2 and 35% by weight).

In certain embodiments, the vesicles may lack a transport enhancing molecule. In some embodiments, the vesicles may lack a "bile acid" such as cholic acid and chenodeoxycholic acid, their conjugation products with glycine or taurine such as glycocholic and taurocholic acid, derivatives including deoxycholic and ursodeoxycholic acid, and salts of each of these acids. In some embodiments, the vesicles may lack acyloxylated amino acids, such as acylcarnitines and salts thereof, and palmitoylcarnitines.

The include an ionic surfactant, e.g., to cause the vesicles to take on a negative charge. The ionic surfactant is dicetyl phosphate (DCP) and will typically comprise, between 1 and 50% by weight of the vesicle-forming lipid (e.g., the non-ionic surfactant). For example, the ionic surfactant(s), if present, may comprise, between 1-5%, 1-10%, 1-15%, 1-20, 1-25%, 1-30%, 1-35%, 1-40%, 1-45%, 5-10%, 5-15%, 5-20%, 5-25%, 5-30%, 5-35%, 5-40%, 5-45%, 5-50%, 10-15%, 10-20%, 10-25%, 10-30%, 10-35%, 10-40%, 10-45%, 10-50%, 15-20%, 15-25%, 15-30%, 15-35%, 15-40%, 15-45%, 15-50%, 20-25%, 20-30%, 20-35%, 20-40%, 20-45%, 20-50%, 25-30%, 25-35%, 25-40%, 25-45%, 25-50%, 30-35%, 30-40%, 30-45%, 30-50%, 35-40%, 35-45%, 35-50%, 40-45%, 40-50%, or 45-50% by weight of the vesicle-forming lipid (e.g., the non-ionic surfactant).

The vesicles include an appropriate hydrophobic material of higher molecular mass that facilitates the formation of bilayers (such as a steroid, e.g., a sterol such as cholesterol). In some embodiments, the presence of the steroid may assist in forming the bilayer on which the physical properties of the vesicle depend. The steroid is cholesterol and will typically comprise between 20 and 120% by weight of the vesicle-forming lipid (e.g., the non-ionic surfactant) (e.g., 20-30%, 20-40%, 20-50%, 20-60%, 20-70%, 20-80%, 20-90%, 20-100%, 20-110%, 30-40%, 30-50%, 30-60%, 30-70%, 30-80%, 30-90%, 30-100%, 30-110%, 30-120%, 40-50%, 40-60%, 40-70%, 40-80%, 40-90%, 40-100%, 40-110%, 40-120%, 50-60%, 50-70%, 50-80%, 50-90%, 50-100%, 50-110%, 50-120%, 60-70%, 60-80%, 60-90%, 60-100%, 60-110%, 60-120%, 70-80%, 70-90%, 70-100%, 70-110%, 70-120%, 80-90%, 80-100%, 80-110%, 80-120%, 90-100%, 90-110%, 90-120%, 100-110%, 100-120%, or 110-120%).

In some embodiments, a lyoprotectant may be included in any solution or mixture prior to lyophilization. Exemplary lyoprotectants include sucrose, trehalose, polyethylene glycol (PEG), dimethyl-succinate buffer (DMS), bovine serum albumin (BSA), mannitol and dextran.

In some embodiments, vesicles of the present disclosure are bilosomes that further include an ionic surfactant or a steroid. In some embodiments, the bilosomes may include both an ionic surfactant and a steroid.

In some embodiments, vesicles of the present disclosure are non-ionic surfactant vesicles **(NISVs)** that lack a transport enhancing molecule and that further include an ionic surfactant or a steroid. In some embodiments, the vesicles may lack a "bile acid" such as cholic acid and chenodeoxycholic acid, their conjugation products with glycine or taurine such as glycocholic and taurocholic acid, derivatives including deoxycholic and ursodeoxycholic acid, and salts of each of these acids. In some embodiments, the vesicles may lack acyloxylated amino acids, such as acylcarnitines and salts thereof, and palmitoylcarnitines. In some embodiments, the **NISVs** may lack a transport enhancing molecule (e.g., any of the aforementioned molecules) and include both an ionic surfactant and a steroid.

### Lyophilization

As discussed herein, in some embodiments, the methods of the present disclosure include a lyophilizing step. Lyophilization involves freezing the preparation in question and then reducing the surrounding pressure (and optionally heating the preparation) to allow the frozen solvent(s) to sublime directly from the solid phase to gas (i.e., drying phase). In certain embodiments, the drying phase is divided into primary and secondary drying phases.

The freezing phase can be done by placing the preparation in a container (e.g., a flask, eppendorf tube, etc.) and optionally rotating the container in a bath which is cooled by mechanical refrigeration (e.g., using dry ice and methanol, liquid nitrogen, etc.). In some embodiments, the freezing step involves cooling the preparation to a temperature that is below the eutectic point of the preparation. Since the eutectic point occurs at the lowest temperature where the solid and liquid phase of the preparation can coexist, maintaining the material at a temperature below this point ensures that sublimation rather than evaporation will occur in subsequent steps.

The drying phase (or the primary drying phase when two drying phases are used) involves reducing the pressure and optionally heating the preparation to a point where the solvent(s) can sublimate. This drying phase typically removes the majority of the solvent(s) from the preparation. It will be appreciated that the freezing and drying phases are not necessarily distinct phases but can be combined in any manner. For example, in certain embodiments, the freezing and drying phases may overlap.

A secondary drying phase can optionally be used to remove residual solvent(s) that was adsorbed during the freezing phase. Without wishing to be bound to any theory, this phase involves raising the temperature to break any physico-chemical interactions that have formed between the solvent molecules and the frozen preparation. Once the drying phase is complete, the vacuum can be broken with an inert gas (e.g., nitrogen or helium) before the lyophilized product is optionally sealed.

### Rehydration

As discussed herein, in some embodiments, the methods of the present disclosure include a step of rehydrating a lyophilized preparation. This is generally achieved by mixing the lyophilized preparation with an aqueous solution. In some embodiments, this involves adding the aqueous solution to the lyophilized preparation.

In some embodiments, the aqueous solution includes a buffer. For example, without limitation, a PCB buffer, an Na₂HPO₄/NaH₂PO₄ buffer, a PBS buffer, a bicine buffer, a Tris buffer, a HEPES buffer, a MOPS buffer, etc. may be used. PCB buffer is produced by mixing sodium propionate, sodium cacodylate, and bis-Tris propane in the molar ratios 2:1:2. Varying the amount of HCl added enables buffering over a pH range from 4-9. In some embodiments, a carbonate buffer may be used. In some embodiments, the aqueous solution is sterile water for injection (WFI).

In some embodiments, a formulation of antigen-containing vesicles prepared by any of the aforementioned methods may be lyophilized for future use and subsequently rehydrated (e.g., with sterile water or an aqueous buffer) prior to use. In some embodiments, an adjuvant may be added during this rehydration step (e.g., by inclusion in the sterile water or aqueous buffer). In some embodiments, a formulation of antigen-containing vesicles may be stored at -80°C prior to lyophilization. In some embodiments, a lyophilized formulation may be stored at a range of temperatures between -20°C and 10°C (e.g., between -5°C and 10°C, between 0°C and 5°C or between 2°C and 8°C).

### Vesicle size and processing

It will be appreciated that a vesicle formulation will typically include a mixture of vesicles with a range of sizes. It is to be understood that the diameter values listed below correspond to the most frequent diameter within the mixture. In some embodiments > 90% of the vesicles in a formulation will have a diameter which lies within 50% of the most frequent value (e.g., 1000 ± 500 nm). In some embodiments the distribution may be narrower, e.g.,> 90% of the vesicles in a formulation may have a diameter which lies within 40, 30, 20, 10 or 5% of the most frequent value. In some embodiments, sonication or ultra-sonication may be used to facilitate vesicle formation and/or to alter vesicle particle size. In some embodiments, filtration, dialysis and/or centrifugation may be used to adjust the vesicle size distribution.

In certain embodiments, the formulation may include vesicles with diameter in range of about 10 nm to about 10 µm. In certain embodiments, vesicles are of diameters between about 100 nm to about 5 µm. In certain embodiments, vesicles are of diameters between about 500 nm to about 2 µm. In certain embodiments, vesicles are of diameters between about 800 nm to about 1.5 µm. In some embodiments, the formulations may include vesicles with a diameter in the range of about 150 nm to about 15 µm. In certain embodiments, the vesicles may have a diameter which is greater than 10 µm , e.g., about 15 µm to about 25 µm. In certain embodiments, the vesicles may have a diameter in the range of about 0.1 µm to about 20 µm, about 0.1 µm to about 15 µm, about 0.1 µm to about 10 µm, about 0.5 µm to about 20 µm, about 0.5 µm to about 15 µm, about 0.5 µm to about 10 µm, about 1 µm to about 20 µm, about 1 µm to about 15 µm, or about 1 µm to about 10 µm. In certain embodiments, the vesicles may have a diameter in the range of about 2 gm to about 10 µm, e.g., about 1 µm to about 4 µm. In certain embodiments, the vesicles may have a diameter which is less than 150 nm, e.g., about 50 nm to about 100 nm.

### Antigens

In general it is to be understood that any antigen or antigens may be entrapped using a method of the present disclosure. As previously discussed, the antigen or antigens may be associated with vesicles in any manner. In some embodiments, the antigen or antigens may be present in the aqueous core of the vesicles. However, depending on its hydrophobicity, an antigen may also be partially or completely associated with a bilayer. In general it is also to be understood that in some embodiments, a vesicle formulation may include amounts of one or more antigens that are not associated with vesicles.

In some embodiments, the methods of the present disclosure may be used to entrap one or more of the antigens included in a vaccine. Table 1 is a non-limiting list of suitable vaccines.

**Table 1**

| **Vaccine** | **Disease** |
|---|---|
| BioThrax^{®} | Anthrax |
| DTaP (Daptacel^{®}, Infanrix^{®}, Tripedia^{®}) | Diphtheria |
| Td (Decavac^{®}) | Diphtheria |
| **DT, TT** | Diphtheria |
| Tdap (Boostrix^{®}, Adacel^{®}) | Diphtheria |
| DTaP/IPV/HepB (Pediarix^{®}) | Diphtheria |
| DTaP/Hib (TriHIBit^{®}) | Diphtheria |
| Hib (ActHIB^{®}, HibTITER^{®}, PedvaxHIB^{®}) | **HIB** |
| HepB/Hib (Comvax^{®}) | **HIB** |
| DTaP/Hib (TriHIBit^{®}) | **HIB** |
| **HPV** (Gardasil^{®}) | **HPV** |
| Influenza (Fluarix^{®}, Fluvirin^{®}, Fluzone^{®}, Flulaval^{®}, FluM1 st^{®}) | Seasonal influenza |
| Influenza (Afluria^{®}) | Seasonal influenza |
| Influenza (Agriflu^{®}) | Seasonal influenza |
| Influenza (Begrivac^{®}) | Seasonal influenza |
| Influenza (Enzira^{®}) | Seasonal influenza |
| Influenza (Fluad^{®}) | Seasonal influenza |
| Influenza (Fluvax^{®}) | Seasonal influenza |
| Influenza (Fluviral, Fluviral **S/F^{®})** | Seasonal influenza |
| Influenza (Grippol^{®}) | Seasonal influenza |
| Influenza (Inflexal, Inflexal S, Inflexal V^{®}) | Seasonal influenza |
| Influenza (Influvac^{®}) | Seasonal influenza |
| Influenza (Mastaflu^{®}) | Seasonal influenza |
| Influenza (Mutagrip^{®}) | Seasonal influenza |
| Influenza (Optaflu^{®}) | Seasonal influenza |
| Influenza (Vaxigrip^{®}) | Seasonal influenza |
| H1N1 pandemic influenza (Arepanrix^{®}) | H1N1 pandemic influenza |
| H1N1 pandemic influenza (Calvapan^{®}) | H1N1 pandemic influenza |
| H1N1 pandemic influenza (Focetria^{®}) | H1N1 pandemic influenza |
| H1N1 pandemic influenza (Influenza A (H1N1) 2009 Monovalent Vaccine^{®}) | H1N1 pandemic influenza |
| H1N1 pandemic influenza (Pandemrix^{®}) | H1N1 pandemic influenza |
| JE (JE-Vax^{®}) | Japanese Encephalitis |
| Lyme Disease (LYMErix^{®}) | Lyme Disease |
| Measles (Attenuvax^{®}) | Measles |
| MMR (M-M-R II^{®}) | Measles |
| MMRV (ProQuad^{®}) | Measles |
| Mening. Conjugate (Menactra^{®}) | Meningococcal |
| Mening. Polysaccharide (Menomune^{®}) | Meningococcal |
| Mumps (Mumpsvax^{®}) | Mumps |
| MMR (M-M-R II^{®}) | Mumps |
| MMRV (ProQuad^{®}) | Mumps |
| DTaP (Daptacel^{®}, Infanrix^{®}, Tripedia^{®}) | Pertussis |
| Tdap (Boostrix^{®}) | Pertussis |
| DTaP/IPV/HepB (Pediarix^{®}) | Pertussis |
| DTaP/Hib (TriHIBit^{®}) | Pertussis |
| Pneumo. Conjugate (Prevnar^{®}) | Pneumococcal |
| Pneumo. Polysaccharide (Pneumovax 23^{®}) | Pneumococcal |
| Polio (Ipol^{®}) | Polio |
| DTaP/IPV/HepB (Pediarix^{®}) | Polio |
| Rabies (BioRab^{®}, Imovax Rabies^{®}, RabAvert^{®}) | Rabies |
| Rotavirus (RotaTeq^{®}) | Rotavirus |
| Rotavirus (Rotarix^{®}) | Rotavirus |
| Rubella (Meruvax II^{®}) | Rubella |
| MMR (M-M-R II^{®}) | Rubella |
| MMRV (ProQuad^{®}) | Rubella |
| Shingles (Zostavax^{®}) | Shingles |
| Vaccinia (Dryvax^{®}) | Smallpox and Monkeypox |
| DTaP (Daptacel^{®}, Infanrix^{®}, Tripedia^{®}) | Tetanus |
| Td (Decavac^{®}) | Tetanus |
| DT, TT | Tetanus |
| Tdap (Boostrix^{®}) | Tetanus |
| DTaP/IPV/HepB (Pediarix^{®}) | Tetanus |
| DTaP/Hib (TriHIBit^{®}) | Tetanus |
| BCG | Tuberculosis |
| Typhoid (Typhim Vi^{®}) | Typhoid |
| Typhoid oral (Vivotif Berna^{®}) | Typhoid |
| Varicella (Varivax^{®}) | Chickenpox (Varicella) |
| MMRV (ProQuad^{®}) | Chickenpox (Varicella) |
| Yellow Fever (YF-Vax •) | Yellow Fever |

In the following sections we discuss some exemplary antigens that could be used.

### Influenza antigens

Influenza is a common infectious disease of the respiratory system associated with the *Orthomyxoviridae* family of viruses. Influenza A and B are the two types of influenza viruses that cause epidemic human disease. Influenza A viruses are further categorized into subtypes on the basis of two surface antigens: hemagglutinin (HA) and neuraminidase (N). Influenza B viruses are not categorized into subtypes. Vaccination is recognized as the single most effective way of preventing or attenuating influenza for those at high risk of serious illness from influenza infection and related complications. The inoculation of antigen prepared from inactivated influenza virus stimulates the production of specific antibodies. Protection is generally afforded only against those strains of virus from which the vaccine is prepared or closely related strains.

Influenza vaccines, of all kinds, are usually trivalent vaccines. They generally contain antigens derived from two influenza A virus strains and one influenza B strain. The influenza virus strains to be incorporated into influenza vaccines each season are determined by the World Health Organization (WHO) in collaboration with national health authorities and vaccine manufacturers. It will be appreciated that any influenza virus strain may be used in accordance with the present disclosure, and that influenza virus strains will differ from year to year based on WHO recommendations.

Monovalent vaccines, which may be useful for example in a pandemic situation, are also encompassed. A monovalent, pandemic flu vaccine will most likely contain influenza antigen from a single A strain. In some embodiments, influenza antigens are derived from pandemic influenza strains. For example, in some embodiments, influenza antigens are influenza A (H1N1 of swine origin) viral antigens.

Predominantly three types of inactivated vaccines are used worldwide to protect against influenza: whole virus vaccines, split virus vaccines containing external and internal components of the virus, and subunit vaccines composed of just external components of the virus (hemagglutinin and neuraminidase). Without wishing to be limited to any theory, it is thought that the higher purity of subunit vaccines should make them less reactogenic and better tolerated. Conversely whole virus and split virus vaccines are thought to contain more epitopes and so be more immunogemc.

In some embodiments, influenza antigens are based on subunit vaccines. Generally, subunit vaccines contain only those parts of the influenza virus that are needed for effective vaccination (e.g., eliciting a protective immune response). In some embodiments, subunit influenza antigens are prepared from virus particles (e.g., purification of particular components of the virus). In some embodiments, subunit influenza antigens are prepared by recombinant methods (e.g., expression in cell culture). For example, US Patent No. 5,858,368 describes methods of preparing a recombinant influenza vaccine using recombinant DNA technology. The resulting trivalent influenza vaccine is based on a mixture of recombinant hemagglutinin antigens cloned from influenza viruses having epidemic potential. The recombinant hemagglutinin antigens are full length, uncleaved, glycoproteins produced from baculovirus expression vectors in cultured insect cells and purified under non-denaturing conditions. In some embodiments, subunit influenza antigens are generated by synthetic methods (e.g., peptide synthesis). Subunit vaccines may contain purified surface antigens, hemagglutinin antigens and neuraminidase antigens prepared from selected strains determined by the **WHO.** Without wishing to be bound by any theories, it is thought that surface antigens, hemagglutinin antigens and neuramidase antigens play a significant role in eliciting production of virus neutralizing antibodies upon vaccination.

In some embodiments, influenza antigens are split virus antigens. Vaccines prepared using split virus antigens typically contain a higher concentration of the most immunogenic portions of the virus (e.g., hemagglutinin and neuramidase), while lowering the concentration of less immunogenic viral proteins as well as non-viral proteins present from eggs (used to produce virus) or extraneous agents (e.g., avian leukosis virus, other microorganisms and cellular debris). Generally, split virus antigens are prepared by a physical process that involves disrupting the virus particle, generally with an organic solvent or a detergent (e.g., Triton X-100), and separating or purifying the viral proteins to varying extents, such as by centrifugation over a sucrose gradient or passage of allantoic fluid over a chromatographic column. In some embodiments, disruption and separation of virus particles is followed by dialysis or ultrafiltration. Split virus antigens usually contain most or all of the virus structural proteins although not necessarily in the same proportions as they occur in the whole virus. Methods of viral splitting as well as suitable splitting agents are known in the art (see for example U.S. Patent Publication No. 20090155309). In some embodiments, final antigen concentration (e.g., of hemagglutinin and/or neuramidase antigens) of split viral antigen is standardized using methods known in the art (e.g., ELISA).

In some embodiments, influenza antigens are whole virus antigens. It is thought that in unprimed individuals, vaccines prepared with whole virus antigens may be more immunogenic and induce higher protective antibody response at a lower antigen dose that other formulations (e.g., subunit or split virus antigens). However, influenza vaccines that include whole virus antigens can produce more side effects than other formulations.

Influenza viral antigens present in immunogenic formulations described herein may be infectious, inactivated or attenuated.

In certain embodiments, an immunogenic formulation may comprise an inactivated viral antigen. It will be appreciated that any method may be used to prepare an inactivated influenza viral antigen. WO 09/029695 describes exemplary methods for producinga whole inactivated virus vaccine. In general, these methods will involve propagating an influenza virus in a host cell, optionally lysing the host cell to release the virus, isolating and then inactivating the viral antigen. Chemical treatment of virus (e.g., formalin, formaldehyde, among others) is commonly used to inactivate virus for vaccine formulation. However, it is to be understood that other techniques could be used, e.g., treatment with chlorine, exposure to high temperatures, etc. In these treatments the outer virion coat is typically left intact while the replicative function is impaired. Non-replicating virus vaccines preferably contain more antigen than live vaccines that are able to replicate in the host.

In certain embodiments, an immunogenic formulation may comprise an attenuated viral antigen. As is well known in the art, one advantage of a vaccine prepared with an attenuated viral antigen lies in the potential for higher immunogenicity which results from its ability to replicate *in vivo* without causing a full infection. Live virus vaccines that are prepared from attenuated strains preferably lack pathogenicity but are still able to replicate in the host. One method which has been used in the art to prepare attenuated influenza viral antigens is viral adaptation which involves serially passing a viral strain through multiple cell cultures. Over time the strain mutates and attenuated strains can then be identified. In certain embodiments the virus may be passed through different cell cultures. In certain embodiments it may prove advantageous to perform one or more of the cell culture steps at a reduced temperature.

Several influenza vaccines are currently licensed (see Table 1). For example, Fluzone^{®}, which is a split cell inactivated influenza vaccine, is developed and manufactured by Sanofi Pasteur, Inc. and may be used in accordance with the present disclosure. Fluzone^{®} contains a sterile suspension prepared from influenza viruses propagated in embryonated chicken eggs. The virus-containing fluids are harvested and inactivated with formaldehyde. Influenza virus is concentrated and purified in a linear sucrose density gradient solution using a continuous flow centrifuge. The virus is then chemically disrupted using a nonionic surfactant, octoxinol-9, (Triton^{®} X-100) producing a split viral antigen. The split virus is then further purified by chemical means and suspended in sodium phosphate-buffered isotonic sodium chloride solution. Fluzone^{®} vaccine is then standardized according to requirements for the influenza season and is formulated to contain 45 µg hemagglutinin (HA) per 0.5 ml dose, in the recommended ratio of 15 µg HA each, representative of the three prototype strains (e.g., 2007-2008 vaccine prepared with A/Solomon Islands/3/2006 (H1N1), A/Wisconsin/67/2005 (H3N2) and B/Malaysia/2506/2004 strains). Fluzone^{®} vaccine is formulated for intramuscular injection.

Another example of a licensed influenza vaccine that may be used in accordance with the present disclosure is Vaxigrip^{®}, which is a split cell inactivated influenza vaccine also developed and manufactured by Sanofi Pasteur, Inc. Vaxigrip^{®} is prepared in a similar fashion to the process outlined above for Fluzone^{®} and is similarly formulated for intramuscular injection.

Yet another example of a licensed influenza vaccine that may be used in accordance with the present disclosure is Flumist^{®}. Flumist^{®} is a live, attenuated trivalent vaccine for administration by intranasal spray. The influenza virus strains in Flumist^{®} have three genetic mutations that lead to temperature restricted growth and an attenuated phenotype. The cumulative effect of the antigenic properties and the genetically modified influenza viruses is that they are able to replicate in the nasopharynx and induce protective immunity. In order to produce Flumist^{®}, specific pathogen-free **(SPF)** eggs are inoculated with each of the appropriate viral strains and incubated to allow vaccine virus replication. The allantoic fluid of these eggs is harvested, pooled and then clarified by filtration. The virus is concentrated by ultracentrifugation and diluted with stabilizing buffer to obtain the final sucrose and potassium phosphate concentrations. Viral harvests are then sterile filtered to produce the monovalent bulks. Monovalent bulks from the three strains are subsequently blended and diluted as requiredto attain the desired potency with stabilizing buffers to produce the trivalent bulk vaccine. The bulk vaccine is then filled directly into individual sprayers for nasal administration. Each pre- filled refrigerated Flumist^{®} sprayer contains a single 0.2 ml dose. Each 0.2 ml dose contains 10^{6.5-7.5}- - FFU of live attenuated influenza virus reassortants of each of the appropriate three viralstrains.

As described above, several influenza vaccines are currently licensed. It is to be understood that any one or combination of these licensed influenza vaccines may be combined with a vesicle as described herein to produce an immunogenic formulation. For example, commercial Fluzone^{®} and/or Vaxigrip^{®} may be combined in this manner to produce an active immunogenic formulation. In some embodiments, licensed influenza vaccines are first purified (e.g., to remove alum adjuvant or other reagents in the vaccine). In some embodiments, licensed influenza vaccines are not purified prior to formulation with a vesicle as described herein.

PCT Patent Application No. PCT/US09/47911 describes some other exemplary influenza antigens that could be used in the methods and formulations of the present disclosure. Exemplary influenza antigens have also been described in U.S. Patent Nos. 7,527,800; 7,537,768; 7,514,086; 7,510,719; 7,494,659; 7,468,259; 7,399,840; 7,361,352; 7,316,813; 7,262,045; 7,244,435; 7,192,595; 7,052,701; 6,861,244; 6,743,900; 6,740,325; 6,635,246; 6,605,457; 6,534,065; 6,372,223; 6,344,354; 6,287,570; 6,136,606; 5,962,298; 5,948,410; and 5,919,480.

### Measles, mumps, rubella and varicella antigens

Several attenuated measles, mumps and rubella (MMR) vaccines are currently licensed. For example, M-M-R-II^{®} is developed and manufactured by Merck & Co., Inc. M-M-R-II^{®} contains a sterile lyophilized preparation of (1) Attenuvax^{®} (Measles Virus Vaccine Live) an attenuated line of measles virus, (2) Mumpsvax^{®} (Mumps Virus Vaccine Live) a strain of mumps virus propagated in chick embryo cell culture, and (3) Meruvax n^{®} (Rubella Virus Vaccine Live) an attenuated strain of rubella virus. Each 0.5 mL dose contains not less than 1,000 TCID₅₀ (50% tissue culture infectious dose) of measles virus, not less than 5,000 TCID₅₀ of mumps virus, and not less than 1,000 TCID₅₀ of rubella virus. Upon reconstitution, M-M-R-II^{®} (as with other licensed MMR vaccines) is typically administered subcutaneously. Although one dose of M-M-R-II^{®} in children over 12 months of age generally induces the production of neutralizing antibodies, some patients fail to seroconvert after the first dose. Accordingly, a second booster is recommended, especially prior to elementary school entry, in order to seroconvert those who did not respond to the first dose.

Another example of an MMR vaccine, PROQUAD^{®} which also contains a Varicella component has been licensed and sold in the Unites States by Merck, although production is currently suspended. PROQUAD^{®} is administered once in children over 12 months of age, with an optional booster administered at least three months later.

It is to be understood that immunogenic compositions provided by the present disclosure may include one or more antigens of an MMR vaccine (e.g., measles, mumps, or rubella virus, or a combination thereof). In some embodiments, immunogenic compositions include a varicella virus (e.g., alone, such as with VARIVAX^{®}, or in combination with other viruses, such as with PROQUAD^{®}).

As is well known in the art, the advantage of using an attenuated virus lies in the potential for higher immunogenicity which results from its ability to replicate *in vivo* without causing a full infection. One method which has been used in the art to prepare attenuated viruses is viral adaptation which involves serially passing a viral strain through multiple cell cultures. Over time the strain mutates and attenuated strains can then be identified. For example, in preparing M-M-R-II^{®}, an attenuated strain of measles virus is propagated in chick embryo cell culture, a B level strain of mumps is propagated in chick embryo cell culture, and an attenuated strain of rubella is propagated in human diploid lung fibroblasts. In certain embodiments the virus may be passed through different cell cultures.

It will be appreciated that any measles, mumps or rubella virus strain may be used, e.g., without limitation any of the following strains which have been described in the art:
- Measles virus Enders' attenuated Edmonston strain (AttA)
- Measles virus attenuated AIK-C strain
- Mumps virus Jeryl Lynn (B-level) strain
- Mumps virus Leningrad Zagreb strain
- Mumps virus Urabe Am 9 strain
- Rubella virus Wistar RA 27/3 strain
- Rubella virus Giguere; 1964 United States
- Rubella virus HPV-77; 1961 United States
- Rubella virus Judith; 1963 Liverpool U.K.
- Rubella virus K0-1; 1967 Kochi, Japan

While all currently licensed MMR vaccines include attenuated viruses, alternative vaccines which include inactivated viruses may also be used in accordance with the present disclosure. In certain embodiments, an immunogenic composition may comprise such an inactivated virus. It will be appreciated that any method may be used to prepare an inactivated virus. In general, these methods will involve propagating a virus in a host cell, lysing the host cell to release the virus, isolating and then inactivating the virus. The virus is typically harvested from cell cultures and screened for infectious dosage as well as for the absence of adventitious agents. Chemical treatment of the virus (e.g., formalin, formaldehyde, among others) is commonly used to inactivate the virus. However, it is to be understood that other techniques could be used, e.g., treatment with chlorine, exposure to high temperatures, etc.

### Other antigens

Canine distemper is a disease caused by viral infection by canine distemper virus, which is a paramyxovirus that is closely related to measles virus. Canine distemper virus may cause serious medical conditions affecting a variety of mammalian species including dogs, weasels, skunks, hyenas, raccoons, and non-domestic felines. Canine distemper infection may causes symptoms including fever, anorexia, runny nose, and eye discharge, and commonly leads to complications such as pneumonia and encephalitis. An attenuated canine distemper vaccine has been licensed, including a multivalent DA2PPC vaccine, which protects against canine distemper (D), adenovirus type 2 (A2), parainfluenza (P), canine parvovirus (P) and canine coronavirus (C). It is to be understood that immunogenic compositions provided by the present disclosure may include one or more components of DA2PPC (e.g., a canine distemper virus antigen).

Rotavirus infection leads to rotavirus gastroenteritis, which can be especially severe in infants and young children. Licensed live attenuated vaccines for treatment of rotavirus infection include RotaTeq^{®} and Rotarix^{®}. RotaTeq^{®} is indicated for the prevention of rotavirus gastroenteritis caused by the G1, G2, G3, and G4 serotypes of the virus. RotaTeq^{®} is administered orally in a three-dose series to infants between the ages of 6 to 32 weeks. Each 2 ml dose of RotaTeq^{®} contains a live reassortant virus, containing G1, G2, G3, G4, and PIA and contains a minimum of 2.0-2.8 × 10⁶ infectious units (IU). Rotarix^{®} is indicated for the prevention of rotavirus gastroenteritis caused by G1, G3, G4, and G9 serotypes of the virus. Rotarix^{®} is administered orally in a two-dose series to infants between the ages of 6 weeks and 24 weeks of age. Each 1 ml dose of Rotarix^{®} contains a minimum of 10⁶ CCID₅₀ oflive, attenuated human G1P rotavirus.

Shingles is a viral infection of the nerve roots, which typically causes pain and rash on one side of the body. Shingles is most common in older adults and people with weak immune systems. A licensed virus for treatment of shingles caused by herpes zoster virus infection is Zostavax^{®}, which is a lyophilized preparation of the Oka/Merck strain of live, attenuated varicella-zoster virus. Zostavax^{®} is indicated for subcutaneous administration and is indicated for individuals 60 years of age and older. Each 0.65 ml dose of Zostavax^{®} contains at least 19,400 pfu of live, attenuated virus.

Another example of a licensed live attenuated vaccine is **DRYVAX^{®},** which is a live-virus preparation of vaccinia virus for treatment of smallpox virus infection. DRYVAX^{®} is prepared from calf lymph which is purified, concentrated, and dried by lyophilization. The reconstituted vaccine has been shown to contain not more than 200 viable bacterial organisms per ml. DRYVAX^{®} is intended for multiple-puncture use, i.e., administration of the vaccine into the superficial layers of the skin using a bifurcated needle. Typically, vaccination with DRYVAX^{®} results in viral multiplication, immunity, and cellular hypersensitivity. With the primary vaccination, a papule appears at the site of vaccination on about the 2nd to 5th day. This becomes a vesicle on the 5th or 6th day, which becomes pustular, umbilicated, and surrounded by erythema and induration. The maximal area of erythema is attained between the 8th and 12th day following vaccination (usually the 10th). The erythema and swelling then subside, and a crust forms which comes off about the 14th to 21st day. At the height of the primary reaction known as the Jennerian response, there is usually regional lymphadenopathy and there may be systemic manifestations of fever and malaise. Primary vaccination with DRYVAX^{®} at a potency of 100 million pock-forming units (pfu)/ml has been shown to elicit a 97% response rate by both major reaction and neutralizing antibody response in children.

Yet another example of a licensed live attenuated vaccine is YF-VAX^{®} for treatment of yellow fever virus infections. YF-VAX^{®} is prepared by culturing the 17D strain of yellow fever virus in living avian leukosis virus-free chicken embryos. YF-VAX^{®} is lyophilized and sealed under nitrogen for storage and is reconstituted immediately prior to use. YF-VAX^{®} is formulated to contain not less than 5.04 Log₁₀ pfu per 0.5 ml dose. Typically, immunity to yellow fever develops by the tenth day after primary vaccination with YF-VAX^{®}. Although it has been demonstrated that yellow fever vaccine immunity can persist for at least 30-35 years, and in some cases for life, booster vaccinations are required at intervals of 10 years in order to boost antibody titer.

In certain embodiments, an immunogenic formulation that is prepared in accordance with the methods of the present disclosure may comprise an antigen that is thermolabile. As used herein, the terms "thermolabile antigen" refer to an antigen that loses antigenic integrity when exposed to certain elevated temperatures. In some embodiments, exposure of a thermolabile antigen to elevated temperatures destroys over 20% of the antigenic integrity of the antigen (e.g., over 30%, over 40%, over 50% or more) as measured in an antigenic integrity assay (e.g., an **ELISA)** as compared to the un-manipulated antigen. In certain embodiments, a thermolabile antigen loses antigenic integrity at temperatures above 30°C (e.g., above 35°C, above 40°C, above 45°C, or above 50°C). In some embodiments, storage of a thermolabile antigen at one of these elevated temperatures for more than 3 minutes (e.g., 5 minutes, 10 minutes, 15 minutes or more) destroys over 20% of the antigenic integrity of the antigen (e.g., over 30%, over 40%, over 50% or more) as measured in an antigenic integrity assay (e.g., an **ELISA)** as compared to the un-manipulated antigen. As discussed herein, methods of the present disclosure are particularly beneficial for thermolabile antigens because they can utilize a lower temperature of antigen solution and/or vesicle-forming lipids, allowing for better preservation of antigenic intergrity.

It is to be understood that the present disclosure is not limited to antigens and that, in general, the methods may be used to entrap any substance whether antigenic or non-antigenic, said methods are not part of the invention.

Therefore, in some embodiments, the methods of the present disclosure may be used to entrap one or more polypeptides, polynucleotides or polysaccharides that may or may not be antigenic. Specific classes of substances include, but are not limited to, adjuvants, enzymes, receptors, neurotransmitters, hormones, cytokines, cell response modifiers such as growth factors and chemotactic factors, antibodies, haptens, toxins, interferons, ribozymes, anti-sense agents, plasmids, **DNA,** and **RNA.** In some embodiments the polypeptide may be an antibody or antibody fragment, e.g., a humanized antibody. In some embodiments, these substances are thermolabile in that they convert into degradants under the conditions referenced above in the context of antigens.

In addition, while the methods of the present disclosure are thought to be particularly applicable to thermolabile substances that are sensitive to their chemical and/or physical environment (e.g., biological substances such as microbes, polypeptides, polynucleotides, polysaccharides, etc.) it is to be understood that in some embodiments, the methods may also be used to entrap more stable substances including traditional small molecule therapeutics.

### Adjuvants

In certain embodiments, the methods of the present disclosure may further include a step of adding one or more adjuvants to a vesicle formulation. As is well known in the art, adjuvants are agents that enhance immune responses. Adjuvants are well known in the art (e.g., see "Vaccine Design: The Subunit and Adjuvant Approach", Pharmaceutical Biotechnology, Volume 6, Eds. Powell and Newman, Plenum Press, New York and London, 1995). In some embodiments, an adjuvant may be added once the vesicle formulation (with entrapped antigen) has been prepared. In some embodiments, an adjuvant may be added during the process of preparing the vesicle formulations (e.g., along with vesicle-forming lipids or other vesicle components, along with the antigen or in a dedicated step).

In certain embodiments, an adjuvant is added before antigen is added. In some embodiments, adjuvant is co-melted with vesicle-forming lipids. In some embodiments, a TLR-3 or TLR-4 agonist adjuvant (described below) is co-melted with vesicle-forming lipids. In certain embodiments, an adjuvant is added after an antigen is added. In some embodiments, adjuvant is added along with a lyoprotectant after an antigen is added. In some embodiments, a TLR-3 or TLR-4 agonist adjuvant (described below) is added along with a lyoprotectant after an antigen is added. In some embodiments, the lyoprotectant is sucrose.

Exemplary adjuvants include complete Freund's adjuvant (CFA), incomplete Freund's adjuvant (IFA), squalene, squalane and alum (aluminum hydroxide), which are materials well known in the art, and are available commercially from several sources. In certain embodiments, aluminum or calcium salts (e.g., hydroxide or phosphate salts) may be used as adjuvants. Alum (aluminum hydroxide) has been used in many existing vaccines. Typically, about 40 to about 700 µg of aluminum is included per dose when given IM. For example, Havrix^{®} includes 500 µg of aluminum per dose.

In various embodiments, oil-in-water emulsions or water-in-oil emulsions can also be used as adjuvants. For example, the oil phase may include squalene or squalane and a surfactant. In various embodiments, non-ionic surfactants such as the mono- and di-C₁₂-C₂₄-fatty acid esters of sorbitan and mannide may be used. The oil phase preferably comprises about 0.2 to about 15% by weight of the immunogenic formulation (e.g., about 0.2 to 1%). PCT Publication No. WO 95/17210 describes exemplary emulsions.

The adjuvant designated **QS21** is an immunologically active saponin fractions having adjuvant activity derived from the bark of the South American tree Quillaja Saponaria Molina, and the methods of its production is disclosed in U.S. Patent No. 5,057,540. Semisynthetic and synthetic derivatives of Quillaja Saponaria Molina saponins are also useful, such as those described in U.S. Patent Nos. 5,977,081 and 6,080,725.

TLRs are a family of proteins homologous to the Drosophila Toll receptor, which recognize molecular patterns associated with pathogens and thus aid the body in distinguishing between self and non-self molecules. Substances common in viral pathogens are recognized by TLRs as pathogen-associated molecular patterns. For example, TLR-3 recognizes patterns in double-stranded RNA, TLR-4 recognizes patterns in lipopolysaccharides while TLR-7/8 recognize patterns containing adenosine in viral and bacterial RNA and DNA. When a TLR is triggered by such pattern recognition, a series of signaling events occurs that leads to inflammation and activation of innate and adaptive immune responses. A number of synthetic ligands containing the molecular patterns recognized by various TLRs are being developed as adjuvants and may be included in an immunogenic formulation as described herein.

For example, polyriboinosinic:polyribocytidylic acid or poly(I:C) (available from InvivoGen of San Diego, CA) is a synthetic analog of double-stranded RNA (a molecular pattern associated with viral infection) and an exemplary adjuvant that is an agonist for TLR-3 (e.g., see Field et al., Proc. Natl. Acad. Sci. USA 58:1004 (1967) and Levy et al., Proc. Natl. Acad. Sci. USA 62:357 (1969)). In some embodiments, poly(I:C) may be combined with other agents to improve stability (e.g., by reducing degradation via the activity of RNAses). For example, U.S. Patent Nos. 3,952,097; 4,024,241 and 4,349,538 describe poly(I:C) complexes with poly-L-lysine. The addition of poly-arginine to poly(I:C) has also been shown to reduce degradation via the activity of RNAses. Poly(IC:LC) is a synthetic, double-stranded poly(I:C) stabilized with poly-L-lysine carboxymethyl cellulose. U.S. Patent Publication No. 20090041809 describes double-stranded nucleic acids with one or more than one locked nucleic acid (LNA) nucleosides that can act as TLR-3 agonists. Those skilled in the art will be able to identify other suitable TLR-3 agonist adjuvants.

Attenuated lipid A derivatives (ALD) such as monophosphoryl lipid A (MPL) and 3-deacyl monophosphoryl lipid A (3D-MPL) are exemplary adjuvants that are agonists for TLR-4. ALDs are lipid A-like molecules that have been altered or constructed so that the molecule displays lesser or different of the adverse effects of lipid A. These adverse effects include pyrogenicity, local Shwarzman reactivity and toxicity as evaluated in the chick embryo 50% lethal dose assay (CELD₅₀). MPL and 3D-MPL are described in U.S. Patent Nos. 4,436,727 and 4,912,094, respectively. MPL was originally derived from lipid A, a component of enterobacterial lipopolysaccharides (LPS), a potent but highly toxic immune system modulator. 3D-MPL differs from MPL in that the acyl residue that is ester linked to the reducing-end glucosamine at position 3 has been selectively removed. It will be appreciated that MPL and 3D-MPL may include a mixture of a number of fatty acid substitution patterns, i.e., heptaacyl, hexaacyl, pentaacyl, etc., with varying fatty acid chain lengths. Thus, various forms of MPL and 3D-MPL, including mixtures thereof, are encompassed by the present disclosure.

In some embodiments these ALDs may be combined with trehalosedimycolate (TDM) and cell wall skeleton (CWS), e.g., in a 2% squalene/Tween^{™} 80 emulsion (e.g., see GB Patent No. 2122204). MPL is available from Avanti Polar Lipids, Inc. of Alabaster, AL as PHAD (phosphorylated hexaacyl disaccharide). Those skilled in the art will be able to identify other suitable TLR-4 agonist adjuvants. For example, other lipopolysaccharides have been described in PCT Publication No. WO 98/01139; U.S. Patent No. 6,005,099 and EP Patent No. 729473.

### II. Methods for Preparing Vesicles - Solvent Injection

In another aspect, the present invention provides methods for preparing vesicles that utilize solvent injection. For example, in some embodiments, the methods involve providing a solution of vesicle-forming lipids and adding the solution of vesicle-forming lipids to an aqueous solution comprising an antigen by injection such that antigen-containing vesicles are formed.

Solvent injection methods may offer some advantages over other vesicle preparation methods, e.g., those methods involving high temperature or pressure methods, since the lipids may be dissolved in organic solutions under temperature controlled conditions. Furthermore, high pressure homogenization can be avoided using solvent injection methods. Indeed, various solvent injection methods have been investigated for preparation of vesicles and have been shown not to require a high temperature during the addition of a lipid-containing solution to an aqueous solution.

In general, it is to be understood that these solvent injection methods may utilize any of the vesicle forming lipids, antigen and adjuvants that were described above for the inverted melt methods of vesicle formation. It is also to be understood that once a composition of antigen-containing vesicles has been prepared by a solvent injection method the composition may be further processed by any one of the lyophilization methods, rehydration methods, vesicle size and processing methods that were described above for the inverted melt methods of vesicle formation.

In certain embodiments, the mixture produced by injecting the solution of vesicle-forming lipids into the aqueous solution comprising an antigen is placed under temperature-controlled conditions of less than 55°C, e.g., less than 50°C, less than 45°C, less than 40°C, less than 35°C, less than 30°C, less than 25°C or even less than 20°C. In certain embodiments, the mixture produced by injecting the solution of vesicle-forming lipids into the aqueous solution comprising an antigen is placed under temperature-controlled conditions in the range of 20-55°C, e.g., 20-50°C, 20-40°C, 20-30°C, 30-55°C, 30-50°C, 30-40°C, 40-55°C, 40-50°C, or 50-55°C. It is to be understood that terms "temperature-controlled conditions" does not require the temperature to be fixed at a particular temperature but simply that the temperature remain within a range (e.g., ±1°C, ±2°C, ±5°C, ±10°C, etc. from some value) or that the temperature remain below or above a particular temperature.

In certain embodiments, the aqueous solution comprising an antigen is at a temperature of less than 50°C prior to injection of the solution of vesicle-forming lipids, e.g., less than 45°C, less than 40°C, less than 35°C, less than 30°C, less than 25°C or even less than 20°C. In certain embodiments, the aqueous solution comprising an antigen is a temperature in the range of 20-60°C, e.g., 20-50°C, 20-40°C, 20-30°C, 30-60°C, 30-50°C, 30-40°C, 30-35°C, 40-60°C, or 40-50°C prior to injection of the solution of vesicle-forming lipids. In certain embodiments, the aqueous solution comprising an antigen is placed under temperature-control prior to injection of the solution of vesicle-forming lipids.

In certain embodiments, the solution of vesicle-forming lipids is at a temperature of less than 90°C when it is injected into the aqueous solution comprising an antigen, e.g., less than 80°C, less than 70°C, less than 65°C, less than 60°C, or less than 55°C. In certain embodiments, the solution of vesicle-forming lipids is at a temperature in the range of 50-90°C when it is injected into the aqueous solution comprising an antigen, e.g., 50-80°C, 50-70°C, 50-650C, 50-60°C, 50-55°C, 55-80°C, 55-70°C, 55-65°C, or 55-60°C.

Various solvent injection methods have been disclosed and can be adapted in accordance with the present disclosure. For example, solvent injection methods in which lipids were dissolved in diethyl ether are discussed in Syan et al. (Nanoparticle vesicular systems: A versatile tool for drug delivery, J. Chemical and Pharmaceutical Research 2(2):496, 2010). In another example, solvent injection methods in which lipids were dissolved in ethanol are discussed in Wagner et al. (Liposome Technology for Industrial Purposes, J. Drug Delivery; Volume 2011, Article ID 591325). In a further example, tert-butyl alcohol was used to dissolve lipids as described by Wang et al. (Colloids and Surfaces V: Biointerfaces 79:254, 2010). See also the methods in Schubert M.A. et al. (European Journal of Pharmaceutics and Biopharmaceutics 55:125-131, 2003).

Vesicle-forming lipids are generally prepared by dissolving lipids in an organic solvent. In some embodiments, the solvent is an ether solvent, e.g., diethyl ether. In some embodiments, the solvent is a polar-protic water-miscible organic solvent. Protic solvents are solvents that contain dissociable protons (e.g., a hydrogen atom bound to an oxygen as in a hydroxyl group or a nitrogen as in an amine group). In some embodiments, the polar-protic water-miscible organic solvent is an aliphatic alcohol having 2-5 carbon atoms (e.g., 2 carbon atoms, 3 carbon atoms, 4 carbon atoms, or 5 carbon atoms). In some embodiments, the solvent is tert-butanol. In some embodiments, the solvent is ethanol.

In some embodiments, the vesicle-forming lipids are dissolved in a polar-protic water-miscible organic solvent without any co-solvents present. In some embodiments, the vesicle-forming lipids are dissolved in a polar-protic water-miscible organic solvent with one or more co-solvents present. In some embodiments one or more of the co-solvents are also polar-protic water-miscible organic solvents. In some embodiments, the polar-protic water-miscible organic solvent makes up at least 70% v/v of the solvent system, e.g., at least 75%, 80%, 90%, 95% or 99%. In some embodiments, the vesicle-forming lipids are dissolved in a water-free solvent system. In some embodiments, the vesicle-forming lipids are dissolved in a solvent system that includes an amount of water such that vesicles do not form. In some embodiments, the vesicle-forming lipids are dissolved in a solvent system that includes less than 5% v/v water, e.g., less than 4%, 3%, 2%, 1%, 0.5%, or 0.1%.

### III. Vesicle formulations

In another aspect, the present disclosure provides antigen-containing vesicle formulations prepared using these methods.

Immunogenic vesicle formulations are useful for treating many diseases in humans including adults and children. In general however they may be used with any animal. In certain embodiments, the methods herein may be used for veterinary applications, e.g., canine and feline applications. If desired, the methods herein may also be used with farm animals, such as ovine, avian, bovine, porcine and equine breeds.

Immunogenic vesicle formulations described herein will generally be administered in such amounts and for such a time as is necessary or sufficient to induce an immune response. Dosing regimens may consist of a single dose or a plurality of doses over a period of time. The exact amount of antigen to be administered may vary from patient to patient and may depend on several factors. Thus, it will be appreciated that, in general, the precise dose used will be as determined by the prescribing physician and will depend not only on the weight of the patient and the route of administration, but also on the frequency of dosing, the age of the patient and the severity of the symptoms and/or the risk of infection. Lower doses of antigen may be sufficient when using an adjuvant. Higher doses may be more useful when given orally, especially in the absence of adjuvants.

In general, the formulations may be administered to a patient by any route. In certain embodiments, the immunogenic formulations may be administered orally (including buccally, sublingually and by gastric lavage or other artificial feeding means). Such oral delivery may be accomplished using solid or liquid formulations, for example in the form of tablets, capsules, multi-particulates, gels, films, ovules, elixirs, solutions, suspensions, etc. In certain embodiments, when using a liquid formulation, the formulation may be administered in conjunction with a basic formulation (e.g., a bicarbonate solution) in order to neutralize the stomach pH. In certain embodiments, the basic formulation may be administered before and/or after the immunogenic formulation. In certain embodiments, the basic formulation may be combined with the immunogenic formulation prior to administration or taken at the same time as the immunogenic formulation. In certain embodiments, the vesicles of an orally administered formulation of the present disclosure may be bilosomes. In certain embodiments, an orally administered formulation of the present disclosure may include a TLR-3 or TLR-4 agonist adjuvant.

In certain embodiments, an immunogenic formulation may also be formulated for delivery parenterally, e.g., by injection. In such embodiments, administration may be, for example, intravenous, intramuscular, intradermal, or subcutaneous, or via by infusion or needleless injection techniques. For such parenteral administration, the immunogenic formulations may be prepared and maintained in conventional lyophilized formulations and reconstituted prior to administration with a pharmaceutically acceptable saline solution, such as a 0.9% saline solution. The pH of the injectable formulation can be adjusted, as is known in the art, with a pharmaceutically acceptable acid, such as methanesulfonic acid. Other acceptable vehicles and solvents that may be employed include Ringer's solution and U.S.P. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The injectable formulations can be sterilized, for example, by filtration through a bacterial-retaining filter, or by incorporating sterilizing agents in the form of sterile solid formulations which can be dissolved or dispersed in sterile water or other sterile injectable medium prior to use.

The immunogenic formulations can also be administered intranasally or by inhalation and are conveniently delivered in the form of a dry powder inhaler or an aerosol spray presentation from a pressurized container, pump, spray, atomiser or nebuliser, with or without the use of a suitable propellant, e.g., dichlorodifluoromethane, trichlorofluoromethane, dichlorotetrafluoroethane, a hydrofluoroalkane, carbon dioxide or other suitable gas. In the case of a pressurized aerosol, the dosage unit may be determined by providing a valve to deliver a metered amount. The pressurized container, pump, spray, atomiser or nebuliser may contain a solution or suspension of the antibody, e.g., using a mixture of ethanol and the propellant as the solvent, which may additionally contain a lubricant, e.g., sorbitantrioleate. Capsules and cartridges (made, for example, from gelatin) for use in an inhaler or insufflator may be formulated to contain a powder mix of the immunogenic formulation and a suitable powder base such as lactose or starch.

Formulations for rectal administration are preferably suppositories which can be prepared by mixing the immunogenic formulation with suitable non-irritating excipients or carriers such as cocoa butter, polyethylene glycol or a suppository wax which are solid at ambient temperature but liquid at body temperature and therefore melt in the rectal vault and release the antibodies. Retention enemas and rectal catheters can also be used as is known in the art. Viscosity-enhancing carriers such as hydroxypropyl cellulose are also certain carriers of the disclosure for rectal administration since they facilitate retention of the formulation within the rectum. Generally, the volume of carrier that is added to the formulation is selected in order to maximize retention of the formulation. In particular, the volume should not be so large as to jeopardize retention of the administered formulation in the rectal vault.

### Formulations

The present invention is limited to formulations consisting of monopalmitoyl glycerol (MPG), cholesterol (CHO) and dicetyl phosphate (DCP), at a ratio of 5:4:1 wherein the molten mixture of vesicle-forming lipids is melted at a temperature of less than 120°C; the formulations further comprising an antigen consisting of an attenuated virus or an inactivated virus, and a suitable buffer.

In some embodiments, the present disclosure provides immunogenic formulations that include an antigen, a TLR-3 agonist adjuvant and a vesicle which comprises a non-ionic surfactant and a transport enhancer which facilitates the transport of lipid-like molecules across mucosal membranes. In some embodiments, these formulations may be administered orally. In some embodiments the TLR-3 agonist adjuvant comprises poly(I:C). In some embodiments the TLR-3 agonist adjuvant comprises poly(IC:LC). In some embodiments, the transport enhancer is a bile acid, a derivative thereof or a salt of any of these (e.g., sodium deoxycholate). In some embodiments, the non-ionic surfactant is a glycerol ester (e.g., 1-monopalmitoyl glycerol). In some embodiments, the vesicle further comprises an ionic amphiphile (e.g., dicetylphospate). In some embodiments, the vesicle further comprises a steroid (e.g., cholesterol). In some embodiments, the vesicles comprise 1-monopalmitoyl glycerol, dicetylphospate, cholesterol and sodium deoxycholate.

In some embodiments, the present disclosure provides immunogenic formulations that include an antigen, a TLR-3 agonist adjuvant and a vesicle which comprises a non-ionic surfactant. In some embodiments, these formulations may be administered parenterally (e.g., by intramuscular injection). In some embodiments the TLR-3 agonist adjuvant comprises poly(I:C). In some embodiments the TLR-3 agonist adjuvant comprises poly(IC:LC). In some embodiments, the non-ionic surfactant is a glycerol ester (e.g., 1-monopalmitoyl glycerol). In some embodiments, the vesicle further comprises an ionic amphiphile (e.g., dicetylphospate). In some embodiments, the vesicle further comprises a steroid (e.g., cholesterol). In some embodiments, the vesicles comprise 1-monopalmitoyl glycerol, dicetylphospate and cholesterol. In some embodiments, the vesicle may lack a transport enhancing molecule. In some embodiments, the vesicle may lack a "bile acid" such as cholic acid and chenodeoxycholic acid, their conjugation products with glycine or taurine such as glycocholic and taurocholic acid, derivatives including deoxycholic and ursodeoxycholic acid, and salts of each of these acids. In some embodiments, the vesicle may lack acyloxylated amino acids, such as acylcarnitines and salts thereof, and palmitoylcarnitines.

In some embodiments, the present disclosure provides immunogenic formulations that include an antigen, a TLR-4 agonist adjuvant and a vesicle which comprises a non-ionic surfactant and a transport enhancer which facilitates the transport of lipid-like molecules across mucosal membranes. In some embodiments, these formulations may be administered orally. In some embodiments the TLR-4 agonist adjuvant comprises monophosphoryl lipid A or 3-deacyl monophosphoryl lipid A. In some embodiments, the transport enhancer is a bile acid, a derivative thereof or a salt of any of these (e.g., sodium deoxycholate). In some embodiments, the non-ionic surfactant is a glycerol ester (e.g., 1-monopalmitoyl glycerol). In some embodiments, the vesicle further comprises an ionic amphiphile (e.g., dicetylphospate). In some embodiments, the vesicle further comprises a steroid (e.g., cholesterol). In some embodiments, the vesicles comprise 1-monopalmitoyl glycerol, dicetylphospate, cholesterol and sodium deoxycholate.

In some embodiments, the present disclosure provides immunogenic formulations that include an antigen, a TLR-4 agonist adjuvant and a vesicle which comprises a non-ionic surfactant. In some embodiments, these formulations may be administered parenterally (e.g., by intramuscular injection). In some embodiments the TLR-4 agonist adjuvant comprises monophosphoryl lipid A or 3-deacyl monophosphoryl lipid A. In some embodiments, the non-ionic surfactant is a glycerol ester (e.g., 1-monopalmitoyl glycerol). In some embodiments, the vesicle further comprises an ionic amphiphile (e.g., dicetylphospate). In some embodiments, the vesicle further comprises a steroid (e.g., cholesterol). In some embodiments, the vesicles comprise 1-monopalmitoyl glycerol, dicetylphospate and cholesterol. In some embodiments, the vesicle may lack a transport enhancing molecule. In some embodiments, the vesicle may lack a "bile acid" such as cholic acid and chenodeoxycholic acid, their conjugation products with glycine or taurine such as glycocholic and taurocholic acid, derivatives including deoxycholic and ursodeoxycholic acid, and salts of each of these acids. In some embodiments, the vesicle may lack acyloxylated amino acids, such as acylcarnitines and salts thereof, and palmitoylcarnitines.

### IV. Kits

In yet another aspect, the present disclosure provides kits that include any lyophilized antigen-containing vesicle formulation of the present disclosure in a first container and an aqueous solution (optionally containing an adjuvant) in a second container. In some embodiments, the kit also includes instructions for mixing the contents of the two containers in order to rehydrate the antigen-containing vesicle formulation.

In some embodiments, the kit may include additional components such as a syringe for injecting the antigen-containing vesicle formulation into a patient.

### Examples

The following examples describe some exemplary modes of making and practicing certain formulations that are described

### Example 1: Lyophilized Influenza Formulations

This Example describes different methods that were used to prepare lyophilized influenza formulations for immunogenicity testing via intramuscular (IM) injection.

### Liposomal Chloroform Method

As used in the following Examples, the liposomal chloroform (CHC1₃) method (described in US Patent No. 5,910,306 to Alving et al.) involved the following steps. A 9:7.5:1 molar ratio of the following lipids: 1,2-ditetradecanoyl-sn-glycero-3-phosphocholine (DMPC), cholesterol (CHO) and 1,2-ditetradecanoyl-sn-glycero-3-phospho-(1'-rac-glycerol) (DMPG) was placed in the bottom of a round bottom glass flask (170.09 mg DMPC, 81.74 mg CHO, 19.64 mg DMPG). Four to ten milliliters of chloroform was added to dissolve the lipids with occasional swirling for 10-20 minutes. Additional chloroform (1 mL) was used to rinse the container. The flask was placed in the rotary evaporator to rotate at 80 rpm at 45°C for 48 to 120 minutes. Solvent was removed by using a vaccum pump until a dry lipid thin film was formed that coated the round flask bottom. After evaporation, the round flask was removed from the rotary evaporator, covered with tissue paper and placed in a dessicator for at least 18 hours using maximum vaccum pressure. The flask with dried thin film was removed from the dessicator. Fluzone^{®} (2009-2010 season) (Sanofi Pasteur) and concentrated phosphate buffer were added together for 5 minutes at 30°C-35°C in a heated water bath (15 ml Fluzone^{®} and 0.289 ml phosphate buffer). Fluzone^{®} (2009-2010 season) (Sanofi Pasteur) is an inactivated vaccine trivalent type A and B (split virion) where each 0.5 ml dose contains 15 µg HA antigen of each of the following influenza virus strains: H1N1, A/Brisbane/59/2007; H3N2, A/Uruguay/716/2007 (A/Brisbane/10/2007-like strain) and B/Brisbane/60/2008. Fluzone^{®} solution and 10-15 glass balls were added to the flask. The liposomal mixture was formed by shaking/incubating the mixture for 8 hours at 220 rpm and 30°C-35°C. An equivalent volume of 400 mM sucrose solution (prepared with sterile water) was added to the liposomal mixture and further shaken for another 5 minutes at 220 rpm at 30°C-35°C. The resulting solution was aliquoted in 1.50 ml aliquots/vial, frozen at -80°C overnight and lyophilized. Lyophilized vials were stored at 4°C for 3 weeks prior to use. Each vial of lyophilized liposome formulated Fluzone^{®} was reconstituted with 0.75 ml of water for injection (WFI) sterile water prior to immunization.

### Melt Method

As used in the following Examples, the melt method (described in US Patent No. 5,679,355 to Alexander et al.) involved the following steps. A 5:4:1 molar ratio of the lipids: monopalmitoyl glycerol, cholesterol and dicetyl phosphate were placed in the bottom of a flat bottom glass beaker (119.29 mg MPG, 112.20 mg CHO, 39.02 mg DCP). The lipids were melted in a heated oil bath at 120°C-125°C, with occasional swirling of the beaker. Fluzone^{®} (2009-2010 season) (Sanofi Pasteur) and concentrated phosphate buffer were added together for 5 minutes at 60°C in a heated water bath (15 ml Fluzone^{®} and 0.289 ml phosphate buffer). The melted lipid mixture (still in the beaker) was transferred from the 120°C-125°C oil bath to a 60°C water bath and the preheated (60°C) Fluzone^{®} solution was immediately added to the melted lipids and homogenized for 10 minutes at 8000 rpm at 60°C. The **NISV** Fluzone^{®} mixture was then shaken for 2 hours at 220 rpm at 30°C-35°C. An equivalent volume of 400 mM sucrose solution (prepared with sterile water) was added and the mixture was further shaken for another 5 minutes at 220 rpm at 30°C-35°C. The resulting solution was aliquoted in 1.50 ml aliquots/vial, frozen at -80°C overnight and lyophilized. Lyophilized vials were stored at 4°C for 3 weeks prior to use. Each vial of lyophilized NISV formulated Fluzone^{®} was reconstituted with 0.75 ml of WFI sterile water prior to immunization.

### Melt Method -Lower Temperature of Antigen Addition

As used in the following Examples, the melt method with a lower temperature of antigen addition involved the following steps. A 5:4:1 molar ratio of the lipids: monopalmitoyl glycerol (MPG), cholesterol (CHO) and dicetyl phosphate (DCP) were placed in the bottom of a flat bottom glass beaker (119.00 mg MPG, 112.64 mg CHO, 39.13 mg DCP). The lipids were melted in a heated oil bath at 120°C-125°C, with occasional swirling of the beaker. Fluzone^{®} (2009-2010 season) (Sanofi Pasteur) and concentrated phosphate buffer were added together for 5 minutes at 30°C in a heated water bath (15 ml Fluzone^{®} and 0.289 ml phosphate buffer). The melted lipid mixture (still in the beaker) was transferred from the 120°C-125°C oil bath to a 30°C water bath and the preheated (30°C) Fluzone^{®} solution was immediately added to the melted lipids and homogenized for 10 minutes at 8000 rpm at 30°C. The NISV Fluzone^{®} mixture was then shaken for 2 hours at 220 rpm at 30°C-35°C. An equivalent volume of 400 mM sucrose solution (prepared with sterile water) was added and the mixture was further shaken for another 5 minutes at 220 rpm at 30°C-35°C. The resulting solution was aliquoted in 1.50 ml aliquots/vial, frozen at -80°C overnight and lyophilized. Lyophilized vials were stored at 4°C for 3 weeks prior to use. Each vial oflyophilized NISV formulated Fluzone^{®} was reconstituted with 0.75 ml of WFI sterile water prior to immunization.

### Inverted Melt Method

As used in the following Examples, the inverted melt method involved the following steps. A 5:4:1 molar ratio of the lipids: monopalmitoyl glycerol (MPG), cholesterol (CHO) and dicetyl phosphate (DCP) were placed in the bottom of a flat bottom glass beaker (119.14 mg MPG, 112.46 mg CHO, 39.67 mg DCP). The lipids were melted in a heated oil bath at 120°C-125°C, with occasional swirling to the beaker. Fluzone^{®} (2009-2010 season) (Sanofi Pasteur) and concentrated phosphate buffer were added together for 5 minutes at 30°C-35°C in a heated water bath (15 ml Fluzone^{®} and 0.289 ml phosphate buffer). The homogenizer was started to homogenize the Fluzone^{®} vaccine at 8000 rpm, then the melted lipids were immediately transferred into the homogenizing Fluzone^{®}, and the homogenization was continued for 10 minutes at 30°C-35°C. The **NISV** Fluzone^{®} mixture was shaken for 2 hours at 220 rpm at 30°C-35°C. An equivalent volume of 400 mM sucrose solution (prepared with sterile water) was added and the mixture was further shaken for another 5 minutes at 220 rpm at 30°C-35°C. The resulting solution was aliquoted into 1.50 ml aliquots/vial, frozen at -80°C overnight and subsequently lyophilized. Lyophilized vials were stored at 4°C for 3 weeks prior to use. Each vial of lyophilized NISV formulated Fluzone^{®} was reconstituted with 0.75 ml of WFI sterile water prior to immunization.

### Solvent Injection Method

In the Examples, the solvent injection method involved injecting a warmed solvent solution (55-65°C) containing vesicle-forming lipids into an aqueous antigen-containing solution (30-35°C). The solvent injection method was investigated as an alternative process to prepare vesicles that does not require a high temperature during the addition of melted lipids to aqueous antigen solution. The amounts of vesicle-forming lipids and antigen were similar to the amounts used in the inverted melt method; however, instead of melting the lipids in a heated oil bath, the lipids were melted in a warm solvent solution of tert-butyl alcohol (TBA) and then dispersed into an aqueous antigen-containing solution while homogenizing or stirring.

### Example 2: Immunization of mice with Influenza Formulations

The influenza formulations prepared by different methods as described in Example 1 were tested in female BALB/C mice 6-8 weeks old (minimum 8 animals per test group) with unformulated commercial Fluzone^{®} (2009-2010 season) (Sanofi Pasteur) (Group 5) acting as a positive control. The mice were immunized intramuscularly (IM) with 50 µ1 of the rehydrated formulations once on day 0. Blood was collected from all mice in the study groups pre-immunization and then post-immunization (14 days after immunization) to assess humoral immune responses. The study design with the various test formulations is shown in Table 2.

**Table 2**

| **Test Article (n=8)** | **Fluzone^{®} (dose)*** | **Formulation method** | **Vesicle Type** | **Storage Temp.** | **Route (volume)*** | **Immunization Schedule** |
|---|---|---|---|---|---|---|
| 1 | 4.5 µg | Inverted Melt Method | NISVs | 4°c | IM (50µ1) | Day 0 |
| 2 | 4.5 µg | Melt Method | NISVs | 4°c | IM (50µ1) | Day 0 |
| 3 | 4.5 µg | Melt Method - Lower Temp. Antigen Addition | NISVs | 4°c | IM (50µ1) | Day 0 |
| 4 | 4.5 µg | Liposomal Chloroform Method | Phospholipid Liposomes | 4°c | IM (50µ1) | Day 0 |
| 5 | 4.5 µg | Commercial Vaccine Control | -- | 4°c | IM (50µ1) | Day 0 |

| | | | | | | |
|---|---|---|---|---|---|---|
| ^{∗} Content per 50 µl mouse dose (mice received 1/10^{th} the human dose of Fluzone^{®} (2009-2010 season) (Sanofi Pasteur). Fluzone^{®} (2009-2010 season) (Sanofi Pasteur) is an inactivated influenza vaccine trivalent types A and B (split virion). Each 0.5 ml human dose of Fluzone^{®} (2009-2010 season) contains 15 µg HA antigen of each of the following influenza virus strains: H1N1, A/Brisbane/59/2007; H3N2, A/Uruguay/716/2007 (A/Brisbane/10/2007-like strain); and B/Brisbane/60/2008. | | | | | | |

### Example 3: sELISA of HA content of Influenza Formulations

The test articles of Example 2 were prepared for **ELISA** analysis by separating each test article into two portions based on density by centrifugation. Briefly, 100 µ1 duplicate samples were diluted in 4900 µl of NaHCO₃ (pH 7.6). The solutions were centrifuged at 4°C for 10 minutes at 24 K using a fixed angle rotor 50.4 Ti. Antigen in the pellet and the supernatant was then subjected to an antigen extraction procedure. Antigen in the supernatant was directly extracted with 25% Triton X-100 while the pellet was resuspended in NaHCO₃buffer (pH 7.6) and then antigen was extracted with 25% Triton X-100. The extracted samples were rotated for 30 minutes at room temperature, sonicated for 5 seconds, and then rotated again for 10 minutes at room temperature. This procedure was repeated two more times before the samples were used for **ELISA** analysis. Positive controls of the commercial Fluzone^{®} vaccine were also prepared in duplicate as above and one sample of commercial Fluzone^{®} vaccine was analyzed without the extraction procedure.

The antigen content of the test articles and controls was then analyzed by sandwich ELISA (sELISA) as follows. 96 well ELISA plates were coated overnight at 4°C with a coating solution of capture antibody, anti-A/Brisbane H1N1 HA serum diluted 1/500 in carbonate-bicarbonate buffer, pH 9.7. The next morning the coating solution was removed from the plates and then a blocking solution was added (5% FBS in ELISA wash buffer 0.05% Tween 20 in PBS) and the plates were blocked for 1-3 hours at 37°C. After incubation, plates were washed with ELISA wash buffer (0.05% Tween 20 in PBS). The starting dilution of the sample in 5% FBS in ELISA wash buffer 0.05% Tween 20 in PBS was prepared and 7 serial 2-fold dilutions were done. The sample and the standard were added to the 96 well ELISA plates and were incubated for 1.5 hours at 37°C. The plates were washed six times in wash buffer and incubated for 1.0 hour at 37°C with a 1/500 dilution of rabbit polyclonal antibody to influenza H1N1 HA as a primary antibody. The plates were washed six times in wash buffer and incubated for 1.0 hours at 37°C with a 1/10000 dilution of a goat anti-rabbit IgG-Fc HRP conjugated secondary antibody (Bethyl). The plates were washed six times and developed with 100 µl of TMB substrate for 8 min. 100 µl of TMB-Stop solution was added to stop the reaction. Absorbance was read at 450 nm with an ELISA plate reader (Bio-Rad). The OD₄₅₀ readings were determined and the results (raw data) were analyzed using the plate reader software (soft Max). The values of the standard curve were used to calculate the concentration of HA in each sample. The linear part of the standard curve was between 0.1-7.5 ng/ml for each influenza strain related protein. For each sample, the dilution giving a concentration in the range of the linear part of the standard curve was used to calculate the original sample concentration. Total HA content was derived by adding the HA content in the supernatant and the pellet.

The total HA content (H1N1 strain) of the test articles of Example 2 are shown in Table 3.

**Table 3**

| **Formulation Method** | **Total HA Content (H1N1 Strain)** |
|---|---|
| Inverted Melt Method | 80% |
| Melt Method | 38% |
| Melt Method - Lower Temperature of Antigen Addition | 63% |
| Liposomal Chloroform Method | 67% |

The inverted melt method produced the highest total HA (H1N1 strain) of all the test articles. In the inverted melt method the molten lipid mixture is added to the antigen solution while in the two other NISV formulation methods, the antigen solution is added to the molten lipid mixture. The inverted nature of the inverted melt method improves total HA content. The results also show that the temperature of antigen addition is an important variable of the formulation method. Indeed, reducing the temperature of antigen addition in the melt method led to a higher total HA content (63% vs. 38%).

### Example 4: Hemagglutinin Inhibition Assay of Potency of Influenza Formulations

For potency testing, the Hemagglutinin Inhibition (HAI) assay was used to measure immunological responses in animals. The HAI assay is a serological technique used to detect HA antibody in serum resulting from infection or vaccination with influenza virus and HAI titers correlate with protection from influenza in humans. Hemagglutination will not occur in the presence of antibodies which bind to and block virus hemagglutinin. The minimum amount of virus that causes hemagglutination of all the Red Blood Cells (RBCs) in a well is known as one hemagglutinating unit (HAU). If an antiserum is titrated against a given number of HAU, the hemagglutination inhibition (HAI) titre and specificity of the antiserum can be determined. Also, if antisera of known specificity are used to inhibit hemagglutination, the antigenic type of an unknown virus can be determined. Hemagglutination occurs when a virion agglutinates (attaches) to a RBC resulting in the formation of a lattice. If no hemagglutination occurs the RBCs will precipitate at the bottom of the well and form a dot. The HAI titer is expressed as the reciprocal of the highest serum dilution showing complete hemmaglutination using four hemagglutination units. An HAI titer of 1:40 or higher is considered as seroprotective, and a four-fold increase in HAI titers in samples taken after and before vaccination is the minimum increase considered necessary for classification of seroconversion. Results are presented as the inverse of HAI titres and geometric mean (GMT) HAI titres. The HAI assay was performed as follows. Briefly, a series of 2-fold dilutions in PBS of sera from immunized mice were prepared in 96-well V-bottomed plates and incubated at room temperature for 30 min with 50 µl of four hemmaglutinating units of A/Brisbane /59/07 (H1N1) or A/Brisbane /10/2007 (H3N2). Next, 50 µl of chicken RBCs (diluted 0.5% v/v) (Canadian Food Inspection Agency, Ottawa, Canada) was added to all wells on the plate and incubated for 30 minutes at room temperature. The highest dilution capable of agglutinating chicken RBCs was then determined.

In this mouse study we evaluated the potency of the same formulations as in Table 2 of Example 2. Table 4 shows the GMT for HAI titer against H1N1 A/Brisbane/59/07 or H3N2 A/Brisbane/I 0/2007 fourteen days after the first immunization (P1Vd14).

**Table 4**

| **Formulation Method** | **GMT p1Vd14 H1N1 A/Brisbane/59/07** | **GMT p1Vd14 H3N2 A/Brisbane/10/2007** |
|---|---|---|
| Inverted Melt Method | 42 | 48 |
| Melt Method | 34 | 10 |
| Melt Method - Lower Temperature of Antigen Addition | 31 | 50 |
| Liposomal Chloroform Method | 28 | 32 |
| Commercial Vaccine Control | 42 | 82 |

The HAI titer against H1N1 for the group treated with Fluzone^{®} formulated into NISV by the inverted melt method was equivalent to the commercial vaccine control (42) and higher than the groups treated with Fluzone^{®} formulated into NISV by either melt methods (34 and 31) and also higher than the liposomes formulated by the chloroform method (28). The HAI titer against H3N2 for the group treated with Fluzone^{®} formulated into NISV by the inverted melt method (48) was significantly higher than the group treated with Fluzone^{®} formulated into NISV by the melt method (10) and also higher than the liposomes formulated by the chloroform method (32). The HAI titer against H3N2 for the group treated with Fluzone^{®} formulated into NISV by the melt method at a lower temperature of antigen addition (50) was comparable to the group treated with Fluzone^{®} formulated into NISV by the inverted melt method (48). These results further confirm the importance of the formulation method and indicate that some HA strains in trivalent vaccines (H3N2) are more thermolabile than others and as a result are more sensitive to the formulation method used.

### Example 5: Stability Testing of Thermostable Lyophilized Influenza Formulations

The stability of lyophilized influenza formulations (NISVs and liposomes) (e.g., as prepared by the various methods described in Example 1) was evaluated at two storage temperature conditions (5°C ± 3°C and 40°C ± 2°C) for up to 7.5 months. There is no single stability-indicating assay or parameter that profiles the stability characteristics of a biological product. As defined by the FDA (FDA Guidance for Industry. Content and Format of Chemistry, Manufacturing and Controls Information and Establishment Description Information for a Vaccine or Related Product), a stability study for a vaccine should generally test for: potency; physicochemical measurements that are stability indicating; moisture content (if lyophilized); pH; sterility or control of bioburden; pyrogenicity and general safety. Consequently, a stability-indicating profile using a number of assays provides assurance that changes in the identity, purity and potency of the product will be detected.

Potency is the specific ability or capacity of a product to achieve its intended effect and is determined by a suitable *in vivo* or *in vitro* quantitative method. A potency assay for the drug product should be sensitive and specific. An *in vivo* mouse potency assay was used to evaluate the potency of the stored formulated and unformulated immunogenic formulation over time. The formulations were administered by the intramuscular route to mice and their immune response was determined using the HAI assay described in Example 4. Physicochemical, biochemical and immunochemical analytical methodologies were also used to characterize changes in the antigen (e.g., molecular size, charge, hydrophobicity) and to detect any degradants. The formulations were also reconstituted with water and tested for appearance (colour and opacity), dissolution time, particle size distribution (PSD), pH and zeta potential. The stability of reconstituted material was tested over 4-6 hours following reconstitution. At the specified timepoints, the excipients (lipids) in the lyophilized formulations were analyzed for purity and related compounds using HPLC. Moisture content in lyophilized formulations was evaluated using the Karl Fischer assay. The formulations used for the stability study were not sterile. However, the formulation involved heating the lipid excipients to > 100°C and adding the melted lipids to sterile filtered buffer solution containing sterile Fluzone^{®} commercial vaccine product. The formulation processes were performed under low bioburden conditions such as in a lamellar flow hood and using Tyvek sterile bags during lyophilization and back filled using sterile nitrogen. Microbial content could be evaluated using a suitable method at the beginning and end of the stability time points.

The general recommendations, as outlined in the ICH Harmonized Tripartite Guideline: Stability Testing of New Drug Substances and Products. Q1A(R2), were followed during the execution of the Stability Study (hereafter, the Study). Proposed stability indicating tests and temperature regimes of lyophilized thermostable influenza formulations are listed in Table 5.

**Table 5**

| **Test/Assay** | **Time points (month*) and animal experiments** | | | |
|---|---|---|---|---|
| | **T=O** | **T=1** | **T=3** | **T=7.5** |
| **Potency** | X | X | X | X |
| **Appearance** | X | X | X | X |
| **ELISA** | X | X | X | X |
| **PSD** | X | X | X | X |
| **pH** | X | X | X | X |
| **HPLC** | O | O | O | O |

| | | | | |
|---|---|---|---|---|
| ^{∗} Month approximately 4 weeks; T=3, and T=6 indicate proposed dates. X - required test; O - optional test | | | | |

In Table 6 is shown the total HA content (H1N1 strain) of test articles described in Example 2 (HA content determined as described in Example 3), after storage at 4°C and 40°C for 7.5 months.

**Table 6**

| **Formulation Method** | **Total HA Content (H1N1 Strain) 4°C Storage** | **Total HA Content (H1N1 Strain) 40°C Storage** |
|---|---|---|
| Inverted Melt Method | 69.21% | 68.98% |
| Melt Method | 36.82% | 35.46% |
| Melt Method - Lower Temperature of Antigen Addition | 63.95% | 64.83% |
| Liposomal Chloroform Method | 71.95%¹ | 70.15%¹ |
| Commercial Vaccine Control | 100% | 53.99% |

The inverted melt method, melt method with lower temperature of antigen addition and the liposomal chloroform method all produced test articles with equivalent high total HA (H1N1 strain) content. The melt method produced a formulation with a considerably lower total HA (H1N1 strain) content. These results are for test articles stored at 4°C for 7.5 months and are comparable to the results obtained at t=0 and presented in Table 3. In the inverted melt method the molten lipid mixture was added to the antigen solution while in the two other **NISV** formulation melt methods, the antigen solution was added to the molten lipid mixture. The inverted nature of the inverted melt method improves total HA content in comparison to the melt method. The results also show that the temperature of antigen addition is an important variable of the formulation method. Indeed, reducing the temperature of antigen addition in the melt method led to a higher total HA content (63.95% vs. 36.82%). Also eliminating temperature as a variable in the liposomal chloroform method led to a higher total HA content (71.95%). When the test articles were stored at 40°C for 7.5 months, all of the test articles retained their total HA content in comparison to the commercial vaccine, which showed a decrease in total HA content of approximately 50%, indicating that all of the lipid containing formulations were equally thermostable with respect to HA content.

In Table 7 is shown the potency of the formulations of Table 6 (HAI titers determined as described in Example 4), after storage at 4°C and 40°C for 7.5 months. Table 7 shows the GMT for HAI titer against H1N1 A/Brisbane/59/07 or H3N2 A/Brisbane/I 0/2007 fourteen days after the first immunization (PI Vd14) in a mouse study as described in Example 2.

**Table 7**

| **Formulation Method** | **GMT p1Vd14 H1N1 A/Brisbane/59/07** | | **GMT p1Vd14 H3N2 A/Brisbane/10/2007** | |
|---|---|---|---|---|
| | 4°C Storage | 40°C Storage | 4°C Storage | 40°C Storage |
| Inverted Melt Method | 48 | 39 | 22 | 15 |
| Melt Method | 34 | 34 | 10 | 10 |
| Melt Method - Lower Temperature of Antigen Addition | 22 | 14 | 17 | 22 |
| Liposomal Chloroform Method | 14 | 17 | 11 | 13 |
| Commercial Vaccine Control | 31 | 10 | 20 | 10 |

The HAI titer against H1N1 for the group treated with Fluzone^{®} formulated into NISV by the inverted melt method (48) was higher than the commercial vaccine control (31) and higher than the groups treated with Fluzone^{®} formulated into NISV by either melt methods (34 and 22) and also higher than the liposomes formulated by the chloroform method (14). The HAI titer against H3N2 for the group treated with Fluzone^{®} formulated into NISV by the inverted melt method (22) was equivalent to the commercial vaccine (20) and higher than the group treated with Fluzone^{®} formulated into NISV by the melt method (10) and also higher than the liposomes formulated by the chloroform method (11). The HAI titer against H3N2 for the group treated with Fluzone^{®} formulated into NISV by the melt method at a lower temperature of antigen addition (17) was slightly lower to the group treated with Fluzone^{®} formulated into NISV by the inverted melt method (22). These results are for test articles stored at 4°C for 7.5 months and further confirm the importance of the formulation method and indicate that some HA strains in trivalent vaccines (H3N2) are more thermolabile than others, and as a result are more sensitive to the formulation method used. When the test articles were stored at 40°C for 7.5 months all of the test articles retained their HAI titer against H1N1 and against H3N2 in comparison to the commercial vaccine which showed a decrease in HAI titer against H1N1 and against H3N2 of approximately 50% indicating that all of the lipid containing formulations were equally thermostable with respect to potency.

In Table 8 is shown the total HA content (H1N1 strain) of test articles prepared by the Solvent injection method described in Example 1 as an alternative method in comparison to the inverted melt method (HA content determined as described in Example 3), after storage at 4°C and 40°C for 3 months.

**Table 8**

| **Formulation Method** | **Total HA Content (H1N1 Strain) 4°C Storage** | **Total HA Content (H1N1 Strain) 40°C Storage** |
|---|---|---|
| Inverted Melt Method | 94% | 108% |
| Solvent Injection Method | 110% | 88% |
| Commercial Vaccine Control | 100% | 29% |

The inverted melt method and the solvent injection method (investigated as an alternative process to prepare liposomes that does not require a high temperature during the addition of melted lipids to aqueous antigen solution) both produced test articles with equivalent high total HA (H1N1 strain) content. These results are for test articles stored at 4°C for 3 months and are comparable to the results obtained at t=0 (data not shown). In the inverted melt method the molten lipid mixture was added to the antigen solution while in the solvent injection method, the lipids were dissolved in a solvent (similar to the liposomal chloroform method) and dispersed into the aqueous antigen solution. When the test articles were stored at 40°C for 3 months both of the formulations retained their total HA content in comparison to the commercial vaccine, which showed a decrease in total HA content of approximately 70%, indicating that both of the formulations were equally thermostable with respect to HA content.

In Table 9 is shown the potency of the same formulations as in Table 8 (HAI titers determined as described in Example 4), after storage at 4°C and 40°C for 3 months. Potency was determined as described in Example 2 except that a second immunization was given on Day 14. Table 9 shows the GMT for HAI titer against H1N1 A/California/7/2009 or H3N2 A/Perth/16/2009 fourteen days after the second immunization (P2Vd14).

**Table 9**

| **Formulation Method** | **GMT P2Vd14 H1N1 A/Brisbane/59/07¹** | | **GMT P2Vd14 H3N2 A/Brisbane/10/2007²** | |
|---|---|---|---|---|
| | 4°C Storage | 40°C Storage | 4°C Storage | 40°C Storage |
| Inverted Melt Method | 483 | 483 | 250 | 232 |
| Solvent Injection Method | 202 | 155 | 136 | 157 |
| Commercial Vaccine Control | 488 | 12 | 164 | 16 |

The HAI titer against H1N1 for the group treated with Fluzone^{®} formulated into NISV by the inverted melt method (483) was equivalent to the commercial vaccine control (499) and higher than the group treated with Fluzone^{®} formulated into NISV prepared by the solvent injection method (202). The HAI titer against H3N2 for the group treated with Fluzone^{®} formulated into NISV by the inverted melt method (250) was slightly higher compared to the commercial vaccine (164) and higher than the group treated with Fluzone^{®} formulated into NISV prepared by the solvent injection method (136). These results are for test articles stored at 4°C for 3 months and further confirm the importance of the formulation method. These results indicate that some HA strains in trivalent vaccines (H3N2) are more thermolabile than others and, as a result are more sensitive to the formulation method used. When the test articles were stored at 40°C for 3 months, the inverted melt method and solvent injection method test articles retained their HAI titer against H1N1 and against H3N2 in comparison to the commercial vaccine, which showed a significant decrease in HAI titer against H1N1 and against H3N2 of approximately 90%, indicating that the lipid containing formulations were thermostable with respect to potency.

### Example 6: Differential Scanning Calorimetry Analysis of NISVs

Differential Scanning Calorimetry (DSC) is a widely used application in understanding the thermal characteristics of materials. The data obtained by DSC of materials can give a range of thermal properties including phase transitions and heat capacity changes, which are key factors of the drug delivery formulation process which allows temperature changes to specific materials to be studied and their influence on the subsequent formulation. DSC works on the principle of measuring the difference in heat energy of a sample pan against a reference pan under the same program and atmospheric conditions. Initially the lipid components (MPG, CHO and DCP) were analyzed individually in the solid state using a TA Instruments Q200 Thermal Analysis DSC. The individual lipids were placed into aluminum pans ensuring the weight of sample was kept constant to ensure accurate enthalpy data. After the individual lipids were tested, a powder blend prepared at the appropriate ratio of lipids (5:4:1) was also tested to see the overall melting temperature of the lipids together. The method used for DSC had a heating rate of 10°C/min over a temperature range from 0-160°C. To prepare the bilayer vesicles the lipid components, in the powder form, were mixed at the appropriate ratio (asdescribed in Example 1, melt method with reduced temperature of antigen addition but using a mock antigen solution) and melted in an oil bath, and while maintaining the molten lipid mixture an emulsion was created by the addition of 6 ml of 25 mM sodium bicarbonate buffer pH 7.6 (30°C) and homogenised for 10 minutes. No antigens were present. Upon cooling, the NISV formulation was incubated for 2 hours with gentle shaking at 220 rpm.

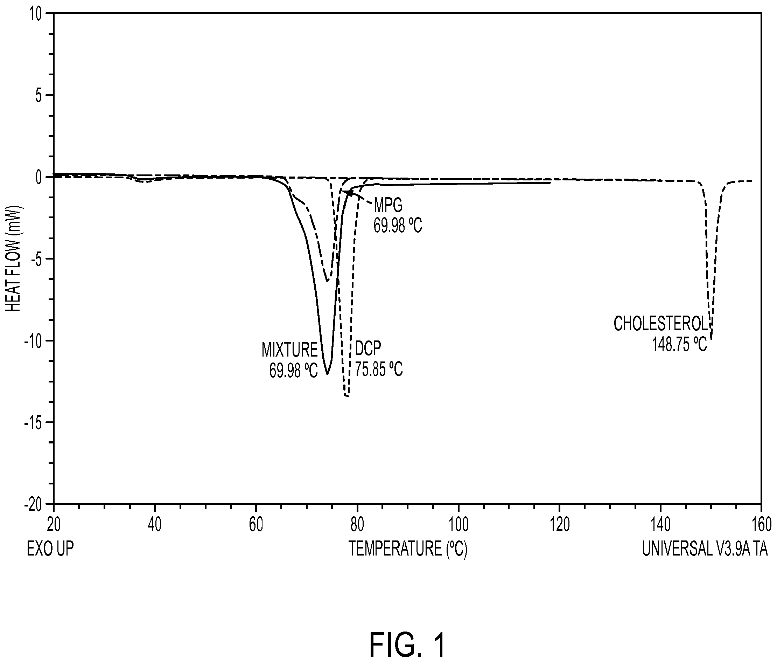

Figure 1 shows the DSC scan of the individual components in the solid state prior to mixing. A broad melting range for each of the lipids can be seen with MPG having the lowest melting point at 69.98 °C, followed by DCP at 75.85 °C and CHO (cholesterol) having the highest melting onset point of 148.75 °C. These are in line with previously reported melting points for the components as stated by the manufacturers. From this initial information it would suggest that to achieve a molten state, the lipids would require heating to approximately 150°C. However, previous protocols (e.g., the melt method as described in Example 1) have suggested that these lipids can be melted by heating to just 120 or 140°C which is below the melting point of cholesterol. However, at this temperature thermogravimetric analysis studies of these lipids under such conditions result in 2.1% weight loss suggesting that heating these lipids to such high temperatures is detrimental. To investigate if a powder blend of the lipids had similar properties to the individual components the lipid mixture was also similarly analysed. From Figure 1 it can be seen that in combination all three lipids melt together with a single main transition at 69.98°C which corresponds to the melting point of MPG. These results suggest that the high melting point cholesterol could be interdigitating with the other two lipids such that the powder blend canbe melted at temperatures of about 95°C when increasing the mass when upscaling from the DSC, i.e., considerably lower than previously reported.

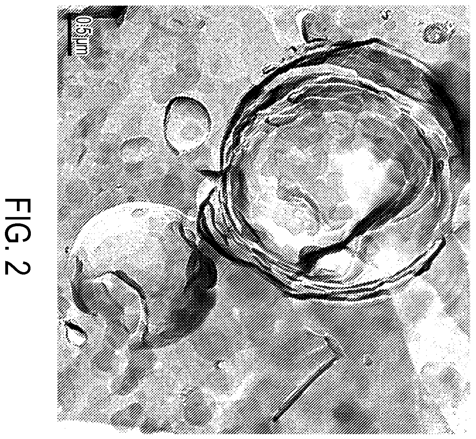

Freeze fracture images were also taken of the NISV formulations to analyze the physical appearance of the NISVs. Small drops of the sample were placed onto ridged, gold specimen or sandwiched between two copper plates supports and were immediately frozen under liquid nitrogen. Fracturing was undertaken on a Balzers apparatus at a temperature of -115°C. The etchings produced were then screened under a transmission electron microscope. Figure 2 shows a freeze fracture image of NISVs showing a large sliced vesicle and a smaller untouched vesicle below it. The scale bar in the lower left corner represents 0.5 µm. Figure 2 confirms the presence of bilayers; it can be seen that the NISVs produced are spherical and multi-lamellar, the layers can be visualized in the bigger vesicle.

### Example 7: Monolayer studies using MPG, CHO and DCP

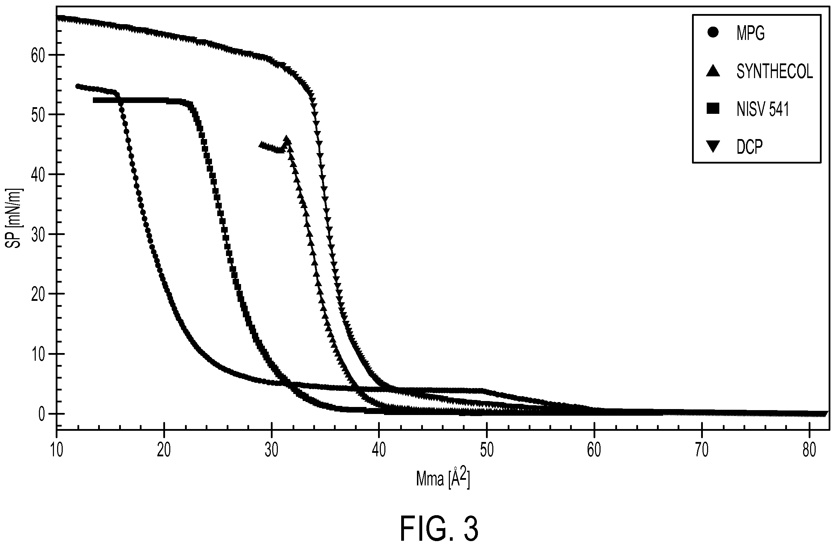

Monolayer studies of the individual lipids (MPG, CHO and DCP) and a mixture of lipids in the ratio 5:4:1 of MPG:CHO:DCP were carried out using a KSV mini trough Langmuir system (KSV Instruments Ltd, Helsinki, Finland) equipped with a platinum Wilhelmy plate in an isolated area. The synthetic cholesterol Synthecol^{™} was used for the CHO components. Filtered double distilled water formed the subphase used in these studies and the temperature of the trough was kept constant at 20 ± 1°C using an external water circulation system. Stock solutions of the individual lipids were prepared at a 0.5 mg/mL in chloroform and a mixture was also prepared in chloroform at the set ratio. 20 µ1 of the lipid stock solutions was spread onto the air/water interface using a glass Hamilton syringe precise to ± 0.2 µ1. Upon spreading of the samples onto the interface the chloroform was left to evaporate and the hydrophilic barriers were set to close at a speed of 10 mm/min to form monolayer isotherms. Each sample was run once until collapse point and then triplicates of the sample were taken using fresh sample. The data was analyzed on the KSV instruments software. Results from the individual monolayer studies (Figure 3) indicate that the ideal lipid mixture of the 5:4:1 (MPG:CHO:DCP) should result in a mean molecular area of 29.2 A²/molecule (Table 10). Table 10 shows the experimental and ideal extrapolated mean molecular area and surface area compression pressure of mixed and pure monolayers at the air/water interface of MPG, CHO, DCP and a mixture of ratio 5:4:1 (MPG:CHO:DCP) (n=3). The experimental value obtained results in a mean molecular area of 28.3 A²/molecule showing a minimal 0.9% deviation from ideality. This data suggests that no lipid is more dominant within the monolayer and that uniform monolayers are favored with an even distribution of the lipids.

**Table 10**

| | Extrapolated area at Zero pressure (A²/molecule) | | Ideal Extrapolated area at Zero pressure (A²/molecule) | Deviation from Ideality | Collapse pressure | |
|---|---|---|---|---|---|---|
| Component | (A²/molecule) | SD | (A²/molecule) | % | mN/m | |
| MPG | 21.2 | 0.4 | - | - | 51.6 | 1.5 |
| Synthecol | 37.1 | 0.4 | | - | 46.6 | 0.5 |
| DCP | 37.5 | 0.5 | - | - | 53.8 | 0.9 |
| Mixture (5:4:1) | 28.3 | 1.5 | 29.2 | 0.9 | 51.9 | 0.1 |

### Example 8: Trypsin Digestion Studies of NISVs

NISV formulations were prepared by the four different methods as described in Example 1. Radiolabeled 1¹²⁵ H1N1 was added to the Fluzone solution as a radioactive tracer. The formulations were subsequently incubated with trypsin (antigen:trypsin weight ratio of 1:2) for 0, 15, 30 and 60 minutes at 37°C. Formulations were then centrifuged twice in double distilled water (100,000 rpm for 40 minutes) to separate unincorporated antigen from associated/incorporated antigen. The resulting pellets (associated/incorporated antigen) were analyzed for radioactivity before and after centrifugation and trypsin digestion.

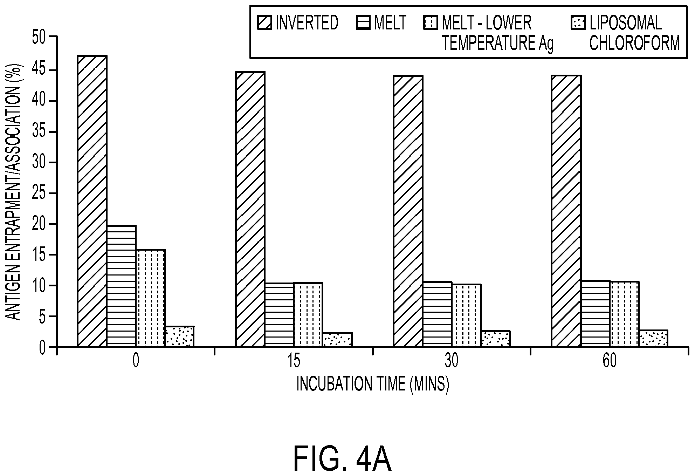

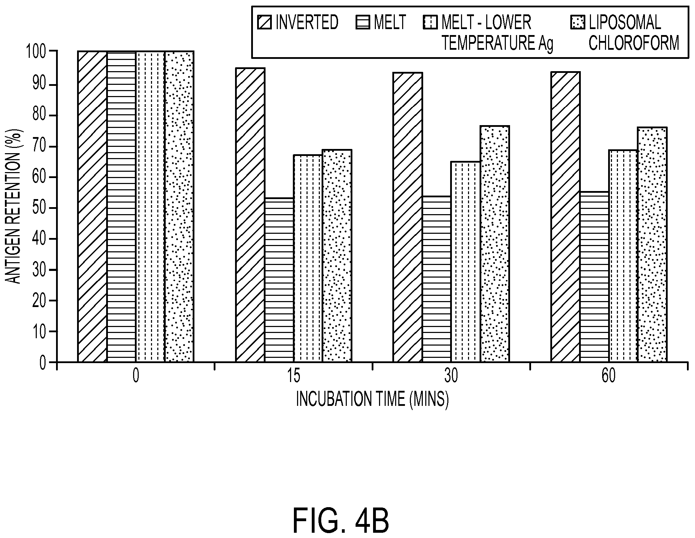

Figure 4 shows the results of a trypsin digestion study on NISVs prepared by the four different formulation methods (i.e., inverted melt method, melt method, melt method - lower temperature of antigen addition and liposomal chloroform method). In Figure 4A, the percent antigen entrapment/association by NISVs (radioactivity in washed pellet) is shown at time 0 (i.e., before trypsin digestion) and then after 15, 30 or 60 minutes of trypsin digestion. The inverted melt method appears to have approximately 2-2.5 fold higher entrapped/associated antigen at time 0 versus the other two melt methods and almost 10 folder higher entrapped/associated antigen in comparison to the liposomal chloroform method. Based on the preliminary studies it was reasoned that entrapped antigen should not be susceptible to trypsin digestion whereas associated antigen would be susceptible to trypsin digestion. Figure 4B shows the same data as Figure 4A except that the values have been normalized to the percentages at time 0. This allows the percent retention of antigen after incubation with trypsin to be more readily visualized. As shown, there is little effect on the percent antigen retention for the inverted melt method formulation, which suggests that the antigen is mostly entrapped and not merely associated where it would be susceptible to tryptic digestion. The melt method shows an approximate 50% decrease in percent antigen retention, which suggests that 50% of the antigen is associated and susceptible to tryptic digestion and 50% of the antigen is entrapped and not susceptible to tryptic digestion. The melt method - lower temperature of antigen addition and liposomal chloroform method show an intermediate effect on the percent of antigen retention which suggests that the predominant portion of antigen is entrapped using these two formulation methods with a lesser amount associated and susceptible to tryptic digestion.

### Example 9: Cryogenic Transmission Electron Microscopy Analysis of NISVs

Cryogenic Transmission Electron Microscopy (Cryo-TEM) was used to analyse NISV formulations prepared as described in Example 1. Preparation of samples for Cryo-TEM was as follows: Freeze dried formulations were removed from storage (2-8°C) and reconstituted with 750 µL distilled water, shaken for 45 seconds and then vigorously vortexed for 1 minute to complete resuspension. 15 µl of the resuspended formulation was applied to both sides of an agar scientific lacey carbon grid which had been glow discharged prior to use. Excess formulation was removed from the grid by blotting with a filter paper. Immediately after blotting, the grid was immersed/quenched in a liquid nitrogen/ethane mixture for 10 seconds and kept in liquid nitrogen until the grid was placed into a Gatan 655 series cryo holder ( 70 degree tilt model also kept immersed under liquid nitrogen conditions). The formulation on the grid was then analysed by a Jeol 2011 LaB6 microscope with a Gatan Ultrascan 1000 camera.

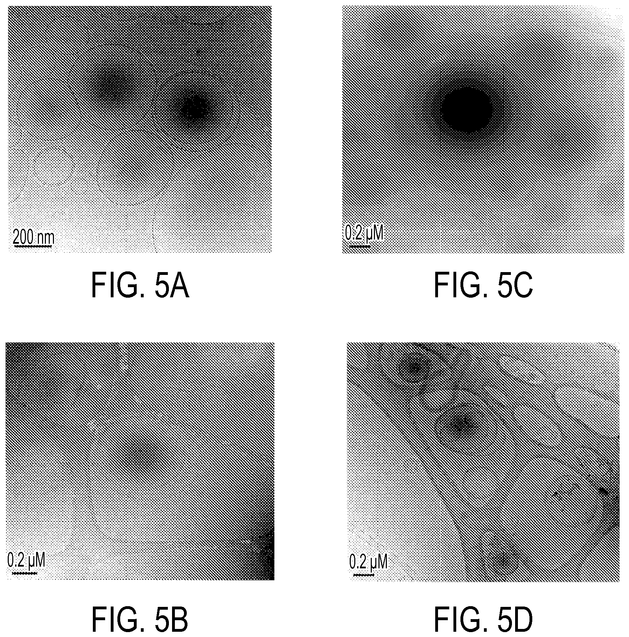

Figure 5 shows cryo-TEM images of vesicles prepared by the (A) inverted melt method, (B) melt method, (C) melt method- lower temperature of antigen addition, and (D) liposomal chloroform method. In general there were a large number of vesicles present across the grid in all four formulations. Dark areas in the cryo-TEM images represent dense spherical vesicles. Darker shading within the vesicles results from the spherical nature of the vesicles; therefore larger vesicles tend to have more curvature and darker staining. The limitation of Cryo-TEM is that during the blotting stage the larger vesicles tend to be removed from the grid onto the blotter, and therefore are not represented. This artifact applies to all four methods of formulation preparation. In Figure SA the inverted melt method with a 10 minute homogenization (as described in Example 1) gives rise to a relatively homogenous population of smaller vesicles; overall the vesicles formed were less multilamellar and more uniform in shape and size. In Figure 5B the melt method gave rise to fewer vesicles overall and not as homogenous a population of vesicles as compared with the inverted melt formulation vesicle population. In Figure *SC* the lower temperature of antigen addition melt method gave rise to a selection of vesicles below 1µm. Similar to the inverted melt method, a greater number of vesicles were apparent in the images in comparison to the melt method formulation. In Figure 4D the liposomal chloroform method produced a variety of vesicles ranging in size from 200 nm up to around 1 micron in size. This formulation method gave rise to large vesicles that were not uniform in shape when compared to the other three formulation methods.

## Claims

1. A method comprising:
providing a molten mixture of vesicle-forming lipids, the mixture consisting of monopalmitoyl glycerol (MPG), cholesterol (CHO) and dicetyl phosphate (DCP), at a ratio of 5:4:1 wherein the molten mixture of vesicle-forming lipids is melted at a temperature of less than 120°C; and
adding the molten mixture of vesicle-forming lipids to an aqueous solution comprising an antigen at a temperature of less than 120°C, said antigen consisting of an attenuated virus or an inactivated virus, and a suitable buffer, wherein said aqueous solution is at a temperature of 30°C to 35°C, and homogenizing the resulting mixture at 30°C to 35°C such that antigen-containing vesicles are formed, wherein said vesicles comprise an aqueous core enclosed by one or more lipid bilayers and preferably further adding a lyoprotectant to the vesicles.

2. The method of claim 1, wherein the antigen is thermolabile.

3. The method of claim 1 or claim 2, further comprising a step of adding an adjuvant after the antigen-containing vesicles are formed, wherein the adjuvant is a TLR-3 agonist.

4. The method of claim 1 or claim 2, wherein the molten mixture of vesicle-forming lipids comprises an adjuvant, wherein the adjuvant is a TLR-4 agonist.

5. The method of any one of claims 1-4, further comprising a step of lyophilizing a formulation that comprises the antigen-containing vesicles.

## Patentansprüche

1. Verfahren, das Folgendes aufweist:
Bereitstellen eines geschmolzenen Gemischs aus vesikelbildenden Lipiden, wobei das Gemisch aus Monopalmitoylglycerol (MPG), Cholesterin (CHO) und Dicetylphosphat (DCP) in einem Verhältnis von 5:4:1 besteht, wobei das geschmolzene Gemisch aus vesikelbildenden Lipiden bei einer Temperatur von weniger als 120 °C geschmolzen wird; und
Zugeben des geschmolzenen Gemischs aus vesikelbildenden Lipiden zu einer wässrigen Lösung, die Folgendes aufweist: ein Antigen bei einer Temperatur von weniger als 120 °C, wobei das Antigen aus einem abgeschwächten Virus oder einem inaktivierten Virus besteht, und einen geeigneten Puffer, wobei die wässrige Lösung eine Temperatur von 30 °C bis 35 °C hat, und Homogenisieren des resultierenden Gemischs bei 30 °C bis 35 °C, sodass antigenhaltige Vesikel ausgebildet werden, wobei die Vesikel einen von einem oder mehreren Lipiddoppelschichten umgebenen wässrigen Kern aufweisen, und vorzugsweise ferner Zugeben eines Lyoschutzmittels zu den Vesikeln.

2. Verfahren nach Anspruch 1, wobei das Antigen thermolabil ist.

3. Verfahren nach Anspruch 1 oder 2, das ferner einen Schritt zum Zugeben eines Adjuvans, nachdem die antigenhaltigen Vesikel ausgebildet wurden, aufweist, wobei das Adjuvans ein TLR-3-Agonist ist.

4. Verfahren nach Anspruch 1 oder 2, wobei das geschmolzene Gemisch aus vesikelbildenden Lipiden ein Adjuvans aufweist, wobei das Adjuvans ein TLR-4-Agonist ist.

5. Verfahren nach einem der Ansprüche 1-4, das ferner einen Schritt zum Lyophilisieren einer Formulierung aufweist, die die antigenhaltigen Vesikel aufweist.

## Revendications

1. Procédé comprenant les étapes consistant à :
fournir un mélange fondu de lipides formant des vésicules, le mélange consistant en monopalmitoyl glycérol (MPG), cholestérol (CHO) et phosphate de dicétyle (DCP), dans un rapport de 5:4 : 1, dans lequel le mélange fondu de lipides formant des vésicules est fondu à une température inférieure à 120°C ; et
ajouter le mélange fondu de lipides formant des vésicules à une solution aqueuse comprenant un antigène à une température inférieure à 120°C, ledit antigène consistant en un virus atténué ou un virus inactivé, et un tampon approprié, ladite solution aqueuse étant à une température de 30°C à 35°C, et
homogénéiser le mélange résultant à 30°C à 35°C de sorte que des vésicules contenant l'antigène soient formées, dans lequel lesdites vésicules comprennent un noyau aqueux enfermé par une ou plusieurs bicouches lipidiques et de préférence en ajoutant en outre un lyoprotecteur aux vésicules.

2. Procédé selon la revendication 1, dans lequel l'antigène est thermolabile.

3. Procédé selon la revendication 1 ou la revendication 2, comprenant en outre une étape consistant à ajouter un adjuvant après la formation des vésicules contenant l'antigène, dans lequel l'adjuvant est un agoniste du TLR-3.

4. Procédé selon la revendication 1 ou la revendication 2, dans lequel le mélange fondu de lipides formant des vésicules comprend un adjuvant, dans lequel l'adjuvant est un agoniste de TLR-4.

5. Procédé selon l'une quelconque des revendications 1 à 4, comprenant en outre une étape de lyophilisation d'une formulation qui comprend les vésicules contenant l'antigène.
